(19) Europäisches Patentamt
European Patent Office
Office européen des brevets

(11) **EP 4 181 148 A1**

(12) **EUROPEAN PATENT APPLICATION**

(43) Date of publication:
**17.05.2023 Bulletin 2023/20**

(21) Application number: **21207844.8**

(22) Date of filing: **11.11.2021**

(51) International Patent Classification (IPC):
***G16B 50/40*** *(2019.01)*

(52) Cooperative Patent Classification (CPC):
**G16B 50/40**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **GARCIA MORCHON, Oscar**
**Eindhoven (NL)**
• **CHEUNG, Yee Him**
**Eindhoven (NL)**

(74) Representative: **Philips Intellectual Property & Standards**
**High Tech Campus 52**
**5656 AG Eindhoven (NL)**

(54) **ENCODING OF DATA IN A HIERARCHICAL DATA STRUCTURE USING HASH TREES FOR INTEGRITY PROTECTION**

(57) Some embodiments are directed to a data structure. The data structure includes multiple blocks and part of a first hash tree. The hash tree is computed from multiple hash values of the multiple genomic blocks. The part of the first hash tree includes at least the first two highest levels of the first hash tree but excluding one or more lower levels of the first hash tree.

Fig. 2a

EP 4 181 148 A1

**Description**

FIELD OF THE INVENTION

The presently disclosed subject matter relates to an encoding system for encoding genomic data in a digital data structure, a verification system for verifying selected genomic data in a digital data structure, an encoding method for encoding genomic data in a digital data structure, a verification method for verifying selected genomic data in a digital data structure, a computer readable medium.

BACKGROUND OF THE INVENTION

As the amount of genomic data is ever-increasing, it is important that such information is stored in an appropriate data structure. ISO/IEC 23092, included herein by reference, defines a standard for encoding, compressing, and protecting genomic data. In particular ISO/IEC DIS 23092-1, "Information technology - Genomic information representation - Part 1:Transport and storage of genomic information", also included herein by reference, defines a data structure for storing and/or streaming of genomic information.

The known data structure discloses a hierarchical data structure in which genomic data, e.g., sequence data, can be stored, associated with other information relating to the genomic data. For example, Table 4 of the ISO/IEC DIS 23092-1 discloses a format structure and hierarchical encapsulation levels. The table shows boxes for various types of data and their possible containment.

Files for genomic data can be very large. Sizes can be hundreds of gigabytes or even terabytes. Conventional integrity measure will take a long time to compute over so much data.

SUMMARY OF THE INVENTION

It would be advantageous to have an improved data structure for genomic data that allows better integrity control. For example, ISO/IEC 23092 mentioned above does not describe a way of grouping all data structures together providing a proof of the integrity of the file efficiently, in particular for the whole file. It is also infeasible to add or remove data structures to a file, or to update genomic files, taking into account the integrity. There is no tracing how the files are updated or who is accountable for those changes. It is advantageous to protect the integrity of genomic data for a long time as it relates not only to the healthcare data of a user, but also of his/her offspring. Using individual digital signatures on selective data components, is not sufficient for this purpose. For example, it does not protect the relationship between data structures. Attacker may remove components or change their order. Any of these problems merit individual addressing. Other issues are identified and addressed herein.

Some embodiments are directed to a digital data structure. The data structure includes multiple genomic blocks and part of a first hash tree. The hash tree is computed from multiple hash values of the multiple genomic blocks. The included part of the first hash tree comprises a selected subset of nodes, which can be a combination of the highest one or multiple levels of nodes and a selected number of leaves of the first hash tree. It is understood that the first hash tree need not be the first hash tree occurring in the data structure.

Genomic data is a particular advantageous application as the data is typically both large and hierarchical. However, embodiment can be applied to any type of data, especially data that is hierarchically organized. Although many embodiments are described in the context of genomic data, the invention is not limited to genomic data.

Generally, a hash tree or Merkle Tree is tree structure in which each of the leaf nodes comprises a hash of a data block of data or the root of a hash tree of a subordinate container, and wherein a non-leaf node comprises a hash over the nodes in the next lower level; the latter nodes may be leaf nodes or non-leaf nodes. A special type of hash tree is Verkle tree; the hash function used to compute an inner node (non-leaf node) from its children is not a regular hash but a vector commitment. Embodiments may use regular hashes, in particular Merkle-Damgård type hash functions (MD-type), such as the SHA family, e.g., SHA-3. The leaves of a Verkle type tree may use a regular hash function, e.g., of MD-type. A further description of Verkle trees can be found in the paper by Kuszmaul, "Verkle Trees", Technical report; Massachusetts Institute of Technology: Cambridge, MA, USA, 2018.The multiple genomic data blocks may have been received, e.g., as a partition of a genomic data. For example, in an embodiment genomic data, such as a genomic sequence and/or other genomic data, is received. The genomic data may already be partitioned in blocks, or may be portioned into blocks by the encoding system.

By including a top level part, quick verification or updating is maintained, but by excluding a lower level part, storage size is reduced. The excluded lower level part may be part of the leaves, all of the leaves, or even several of the lower levels.

In an embodiment, the data structure is a hierarchical data structure. A block at a higher level may refer to multiple blocks at a lower level. The higher level blocks may include part of a hash tree computed over the lower level.

This may happen more than once. For example, a first level may include part of a hash tree computed over blocks at a second lower level. The second level may include part of a hash tree computed over blocks at a third lower level, and so on. The first level may include part of another hash tree computed over other blocks at the second lower level.

The data structure or part thereof may be stored, retrieved, streamed, received, encoded, and verified. When streaming a data structure the excluded parts may be recomputed and included in the streaming. When streaming a data structure, the streaming may only comprise selected genomic data blocks and part of the hash tree needed for recomputing the root of the hash tree without access to the non-streamed data.

In an embodiment, a hash tree is not stored in full. This may also happen across multiple levels. Interestingly, a first level comprises a partial hash tree for a second lower level, while the second level also contains a partial hash tree for a lower third level. A hierarchy of partially included hash tree may be constructed. In an embodiment, a tree parameter is received. The size of the part of the hash tree that is included is determined from the tree parameter. For example, the tree parameter may be the number of levels to include. In an embodiment, the tree parameter is at least two.

Other aspects of the hash tree, e.g., the number of children per nodes, the k-aryness, may also be set by a tree parameter.

An aspect is a verification system for verifying selected data in a data structure, e.g., as encoded by an embodiment of an encoding system. Verification may be done by verifying a root of a hash tree. This is typically done by recomputing the root. Although, for more advanced types of hash trees, e.g., Verkle trees, asymmetric algorithms may be used. To recompute or otherwise verify the hash, part of the hash tree may either be retrieved from the data structure or may be recomputed. For example, leaves values may be absent, and may be recomputed. Some of the lower levels (or part thereof) may also be absent in the data structure and may also be recomputed. However, some hash values may be present in the data structure and can be retrieved. To determine which values are needed, one may identify a path starting from data blocks selected for verification to a root of a corresponding hash tree. The hash values for the hash tree along the path that are needed for precomputation of the root and/or verification may include the values of nodes on the path, but may also include children of nodes on the path. Interestingly, the root may be the root of the hash tree directly associated with the data blocks that are verified, e.g., the hash tree that include hash values of the data blocks in its leaves, e.g., a first hash tree, but may instead (or also) be the root of a hierarchically higher hash tree, e.g., a second hash tree. Once the root has been recomputed it can be compared to the root in the data structure. Other types of verification include verifying a signature over the root, or performing vector commitment verification as in a Verkle tree.

An encoding system may be an electronic system possibly implemented in one or multiple electronic devices. A verification system may be an electronic system possibly implemented in one or multiple electronic devices.

A further aspect is an encoding method and a verification method. An embodiment of the method may be implemented on a computer as a computer implemented method, or in dedicated hardware, or in a combination of both. Executable code for an embodiment of the method may be stored on a computer program product. Examples of computer program products include memory devices, optical storage devices, integrated circuits, servers, online software, etc. Preferably, the computer program product comprises non-transitory program code stored on a computer readable medium for performing an embodiment of the method when said program product is executed on a computer.

In an embodiment, the computer program comprises computer program code adapted to perform all or part of the steps of an embodiment of the method when the computer program is run on a computer. Preferably, the computer program is embodied on a computer readable medium.

BRIEF DESCRIPTION OF THE DRAWINGS

Further details, aspects, and embodiments will be described, by way of example only, with reference to the drawings. Elements in the figures are illustrated for simplicity and clarity and have not necessarily been drawn to scale. In the figures, elements which correspond to elements already described may have the same reference numerals. In the drawings,

Fig. 1a schematically shows an example of an embodiment of an encoding system,
Fig. 1b schematically shows an example of an embodiment of a verification system,
Fig. 2a schematically shows an example of an embodiment of an encoded data format for storage,
Fig. 2b schematically shows an example of an embodiment of an encoded data format for storage,
Fig. 2c schematically shows an example of an embodiment of an encoded data format for storage,
Fig. 3 schematically shows an example of an embodiment of a hierarchical hash tree,
Fig. 4 schematically shows an example of an embodiment of a hash tree,
Fig. 5a schematically shows an example of an embodiment of a hash tree,
Fig. 5b schematically shows an example of an embodiment of a hash tree,
Fig. 6 schematically shows an example of an embodiment of an encoded message for streaming or transport,

Fig. 7a schematically shows an example of an embodiment of an encoding method,
Fig. 7b schematically shows an example of an embodiment of a verification method,
Fig. 8a schematically shows a computer readable medium having a writable part comprising a computer program according to an embodiment,
Fig. 8b schematically shows a representation of a processor system according to an embodiment.

**Reference signs list**

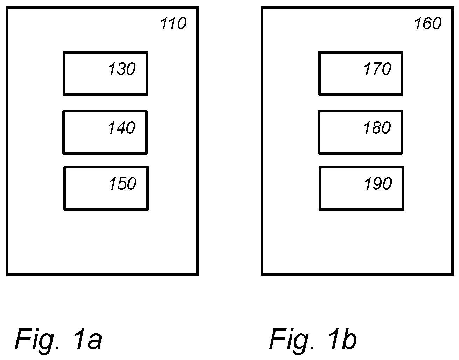

The following list of references and abbreviations corresponds to Figs. 1a-3 and is provided for facilitating the interpretation of the drawings and shall not be construed as limiting the claims.

| | |
|---|---|
| 110 | an encoding system |
| 130 | a processor system |
| 140 | storage |
| 150 | communication interface |
| 160 | a verification system |
| 170 | a processor system |
| 180 | storage |
| 190 | communication interface |
| 200 | an encoding system |
| 210 | a hash tree unit |
| 211-213 | a hash tree encoding |
| A, A1, ..., A34 | hierarchical data |
| 220 | a storage unit |
| 230 | a streaming unit |
| 240 | a verification unit |
| 300 | a hash tree |
| 310 | a highest level |
| 311 | a second-highest level |
| 313 | a third-highest level |
| 320 | a leaves level |
| 330 | a selected part |

| | |
|---|---|
| 1000 | a computer readable medium |
| 1010 | a writable part |
| 1020 | a computer program |
| 1110 | integrated circuit(s) |
| 1120 | a processing unit |
| 1122 | a memory |
| 1124 | a dedicated integrated circuit |
| 1126 | a communication element |
| 1130 | an interconnect |
| 1140 | a processor system |

DETAILED DESCRIPTION OF THE EMBODIMENTS

While the presently disclosed subject matter is susceptible of embodiment in many different forms, there are shown in the drawings and will herein be described in detail one or more specific embodiments, with the understanding that the present disclosure is to be considered as exemplary of the principles of the presently disclosed subject matter and not intended to limit it to the specific embodiments shown and described.
In the following, for the sake of understanding, elements of embodiments are described in operation. However, it will be apparent that the respective elements are arranged to perform the functions being described as performed by them.
Further, the subject matter that is presently disclosed is not limited to the embodiments only, but also includes every other combination of features described herein or recited in mutually different dependent claims.
**Fig. 1a** schematically shows an example of an embodiment of an encoding system 110. **Fig. 1b** schematically shows an example of an embodiment of a verification system 160. Encoding system 110 comprises a processor system 130, a storage 140, and a communication interface 150. Verification system 160 comprises a processor system 170, a storage 180, and a communication interface 190.

For example, communication interface 150 may comprise an input interface configured for receiving genomic data, e.g., a genomic sequence and/or other genomic data. Processor system 130 may be configured, e.g., through software stored in storage 140, to generate a data structure. The data structure comprising multiple genomic blocks and part of the first hash tree. It is noted that, instead of genomic data other types of data may be used, in particular other hierarchical data.

For example, communication interface 190 may comprise an input interface configured receiving a data structure. Processor system 170 may be configured to verify a recomputed root of a first hash tree with the highest level of the first hash tree in the obtained data structure.

Storage 140 and/or 180 may be comprised in an electronic memory. The storage may comprise non-volatile storage. Storage 140 and/or 180 may comprise non-local storage, e.g., cloud storage. In the latter case, the storage may be implemented as a storage interface to the non-local storage.

Systems 110 and/or 160 may communicate with each other, external storage, input devices, output devices, and/or one or more sensors over a computer network. The computer network may be an internet, an intranet, a LAN, a WLAN, etc. The computer network may be the Internet. The systems comprise a connection interface which is arranged to communicate within the system or outside of the system as needed. For example, the connection interface may comprise a connector, e.g., a wired connector, e.g., an Ethernet connector, an optical connector, etc., or a wireless connector, e.g., an antenna, e.g., a Wi-Fi, 4G or 5G antenna. The sensor may be a sequencing device for obtaining genomic sequencing data from a sample.

The systems may be configured for digital communication, which may include, e.g., receiving genomic data, storing the data structure, streaming the data structure, obtaining, e.g., receiving or retrieving the data structure, e.g., for verification.

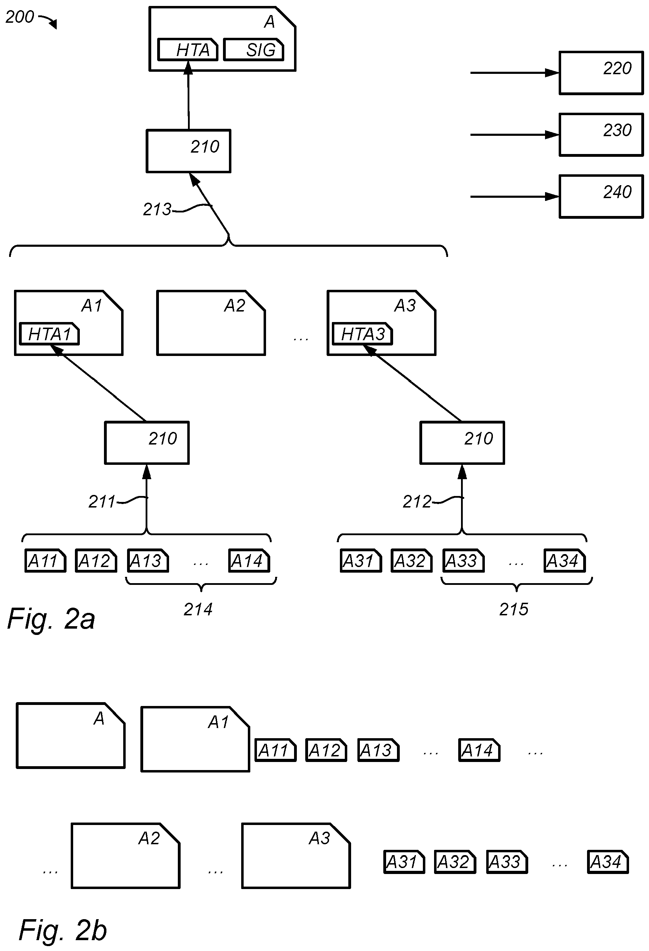

The execution of system 110 and 160 may be implemented in a processor system, e.g., one or more processor circuits, e.g., microprocessors, examples of which are shown herein. Figures and description describe functional units that may be functional units of the processor system. For example, Fig. 2a may be used as a blueprint of a possible functional organization of the processor system. The processor circuit(s) are not shown separate from the units in these figures For example, the functional units shown in may be wholly or partially implemented in computer instructions that are stored at system 110 and 160, e.g., in an electronic memory of system 110 and 160, and are executable by a microprocessor of system 110 and 160. In hybrid embodiments, functional units are implemented partially in hardware, e.g., as coprocessors, e.g., cryptographic coprocessors, and partially in software stored and executed on system 110 and 160.

In the various embodiments of systems 110 and 160, the communication interfaces may be selected from various alternatives. For example, the interface may be a network interface to a local or wide area network, e.g., the Internet, a storage interface to an internal or external data storage, a keyboard, an application interface (API), etc.

The systems 110 and 160 may have a user interface, which may include well-known elements such as one or more buttons, a keyboard, display, touch screen, etc. The user interface may be arranged for accommodating user interaction for configuring the systems, retrieving genomic data, displaying genomic data, verifying genomic data, and the like.

Storage may be implemented as an electronic memory, say a flash memory, or magnetic memory, say hard disk or the like. Storage may comprise multiple discrete memories together making up storage 140, 180. Storage may comprise a temporary memory, say a RAM. The storage may be cloud storage.

System 110 may be implemented in a single device. System 160 may be implemented in a single device. Typically, the system 110 and 160 each comprise a microprocessor which executes appropriate software stored at the system; for example, that software may have been downloaded and/or stored in a corresponding memory, e.g., a volatile memory such as RAM or a non-volatile memory such as Flash. Alternatively, the systems may, in whole or in part, be implemented in programmable logic, e.g., as field-programmable gate array (FPGA). The systems may be implemented, in whole or in part, as a so-called application-specific integrated circuit (ASIC), e.g., an integrated circuit (IC) customized for their particular use. For example, the circuits may be implemented in CMOS, e.g., using a hardware description language such as Verilog, VHDL, etc. In particular, systems 110 and 160 may comprise circuits for the cryptographic functions, such as hash function or signatures.

A processor circuit may be implemented in a distributed fashion, e.g., as multiple sub-processor circuits. A storage may be distributed over multiple distributed sub-storages. Part or all of the memory may be an electronic memory, magnetic memory, etc. For example, the storage may have volatile and a non-volatile part. Part of the storage may be read-only.

Encoding system 110 may be implemented as a single device or as multiple devices. Verification system 160 may be implemented as a single device or as multiple devices.

**Fig. 2a** schematically shows an example of an embodiment of an encoding system 200. Shown in Fig. 2a is a hierarchical data structure having multiple levels. At the highest level, a single data block is shown labeled A. At the second-highest level multiple blocks are shown, in this example, three data blocks are shown, labeled A1, A2, and A3. Two of the data blocks at the second-highest level have yet a further hierarchical level below it. At the third-highest level, two data block sequences are shown: data blocks A11, A12, up to, A14 are subordinate to data block A1; data blocks A31, A32, up to, A34 are subordinate to data block A3. There may be more than three levels in the hierarchical data structure. Below any data block there may be more than four subordinate blocks, e.g., 8 or more, 20 or more, etc.

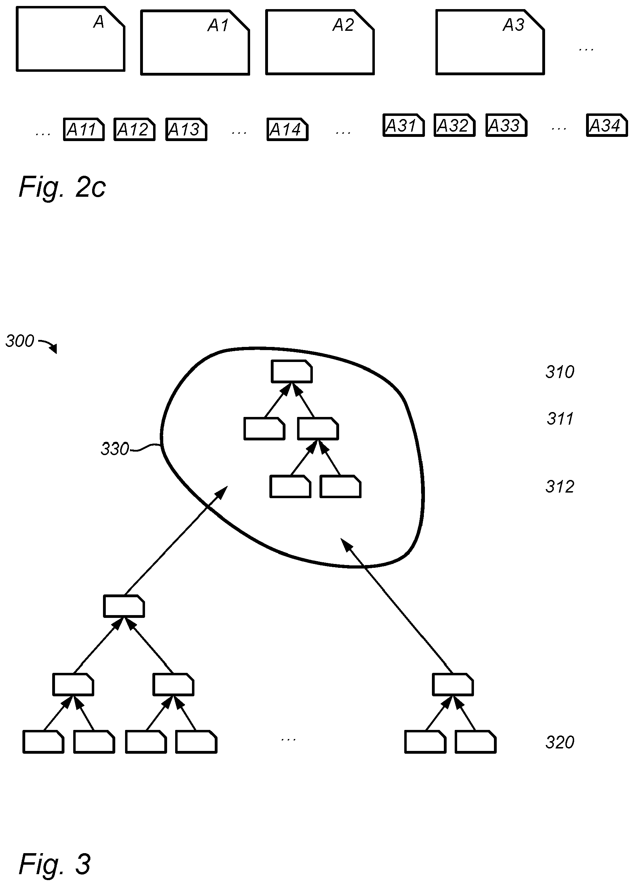

For the multiple blocks in a sequence, a hash tree is constructed. Fig. 2 shows a hash tree unit 210. Hash tree unit may be a functional unit of encoding system 200. Hash tree unit 210 is configured to construct a hash tree for a sequence of data blocks. A hash tree is also known as a Merkle tree. The hash tree may be a binary hash tree. Examples of hash trees are further shown herein, e.g., with reference to Figs. 3, 4, and 5.

For example, data such as genomic data may be portioned into a sequence of multiple genomic data blocks 214. Sequence 214 is shown with 3 blocks, but may often have more than three blocks, e.g., even many more, e.g., more than 10, more than 100, etc. In addition to genomic data the multiple blocks may comprise additional data blocks with additional data, e.g., information associated with the genomic data, e.g., its origin, meaning, purpose, etc. Shown in Fig. 2 are additional blocks A11, A12 and genomic data blocks A13 up to A14; Shown in Fig. 2 are additional blocks A31, A32 and genomic data blocks A33 up to A34.

An input interface may be configured for receiving genomic data, e.g., in the form of data blocks, but may also be configured to receive further data items. For example, the further data items and/or genomic data may be retrieved from multiple files, which are combined in the data structure. The further data items may be assigned to further leaves of the first hash tree, e.g., their hash values may be included in leaves of the hash tree.

A hash tree, also known as a Merkle tree, is a tree in which the leaf nodes are labelled with the cryptographic hash of a data block, and every non-leaf node is labelled with the cryptographic hash of the labels of its child nodes. In this case, the hash tree constructed at 211, may have hashes of blocks A11 up to A14 for its leaves. Most nodes will typically have two children, though one or more nodes may have only one, e.g., to account for a number of blocks that is not a power of two. As a variant, a hash tree could have nodes that have more than three children.

In the hierarchical part of the data structure above sequence A11-A14, part of the hash tree is included. For example, hash tree unit 210 may compute multiple hash values for the multiple genomic blocks A13 up to A14 by applying a hash function at least to the genomic data in the multiple genomic data blocks. The hash function is preferably a cryptographic strong hash function, e.g., SHA-3. Optionally, additional hashes are computed for additional data blocks, e.g., blocks A11 and A12. Hash tree unit 210 computes a first hash tree for the multiple hash values, assigning the multiple hash values to leaves of the first hash tree. In an embodiment, the first hash tree has at least three levels. In an embodiment, the first hash tree has at least four leaves.

The hash function may be a regular hash function, in particular of Merkle-Damgård construction comprising a one-way compression function. The nodes in the hash tree, or Merkle Tree, are not necessarily computed by applying such a hash function. Other types of fingerprint algorithms, such as vector commitments, e.g., for Verkle Trees, or other authentication tags may be used. There may be multiple sequences of data blocks, including multiple sequences of genomic data blocks. Fig. 2 shows a second example of such a sequence: blocks A31-A34. Like for sequence A11-A13, also for these blocks, hash values are computed and a hash tree is constructed.

Interestingly, hash tree information obtained for the hash tree computed for a sequence of data blocks is included in a block that is hierarchically above it. For example, block A1 is hierarchical above the blocks A11-A14. Hash tree information is included in one or more blocks at the higher level. In the example, block A1 comprises hash tree information HTA1. Likewise, in this example, block A3 comprises hash tree information HTA3, obtained from the hash tree computed by hash tree unit 210 for sequence A31-A34.

In an embodiment, hash tree information comprises at least the root of the hash tree, and preferably also the level immediately below it. For example, hash tree information may include the two highest levels of the hash tree. Interestingly, the full hash tree is not included in the hash tree information. A larger or smaller part of the hash tree is excluded. For example, in an embodiment one or more of the lowest levels, starting at the leaves, may be excluded from the hash tree information. Excluding part of the hash tree reduces the storage or streaming requirements for the data structure. The excluded information can be recomputed later should they be needed, e.g., for verification, however, as these levels are close to the leaves, they are computed over relatively little information. For example, to recompute the hash value in a leaf, only the hash over a single data block needs to be computed. Whereas to recompute, say, a node close to the root of the hash tree, many more blocks may need to be included in the computation.

In an embodiment, a lower level hash tree may be included in the data structure in full, while the root of the lower hash tree is used in a leaf of a further hash tree. The further hash tree may be partially included, in particular the leaf comprising the root of the lower hash tree may be omitted. Also other parts of the further hash tree may be omitted, especially one or more of the lower or lowest levels.

By including levels close to the top, e.g., at least the first two levels, though more levels are possible, computation time is reduced the most, while by excluding levels close to the bottom, storage requirements are reduced the most.

Constructing hash trees can be done more than once. For example, Fig. 2a shows another example of a sequence of blocks A31-A34. Hash tree unit 210 may be configured to compute hash values for the blocks, compute a hash tree and include part of the tree, e.g., the first number of levels, say the first two levels, but excluding at least part of the tree, e.g., the lowest or lower levels in the hash tree information for a hierarchical higher block, in this case HTA3.

The blocks comprising hash tree information, in this case blocks A1 and A3 may be dedicated to storing hash tree information The hash tree information may be included along with other information, e.g., information relating to data in het hierarchical lower and/or hierarchical levels.

Constructing hash trees can be done at multiple levels. For example, Fig. 2a shows another example of a sequence of blocks: A1-A3. Blocks A1-A3 are at a hierarchical higher level than blocks A11-A14 and A31-A34. One, or more, or all of these blocks contain hash tree information of hash trees at a lower hierarchical level; in this case blocks A1 and A3. Hash tree unit 213 may compute hash values for the blocks in this sequence, construct a hash tree and include part of the hash tree, e.g., a top part, in a hierarchical higher block; in this case block A. The hash tree information is labeled HTA. There may be multiple sequences at this level two, e.g., a sequence B1, B2, etc. (not shown in Fig. 2a). There may be multiple blocks at the level of block A, e.g., block B, C (not shown in Fig. 2a).

The hash tree information can be used in verification to verify the integrity of the information that was used to compute the hash tree information. Generally speaking it is advantageous to include more data in one or more hash tree so that more data can be verified; preferably all genomic information is used to compute one or more hash trees. However, it may happen that data is included which is not as sensitive as other data, e.g., reference data, instruction data, etc. Some parts of the genome are considerably more sensitive than others, e.g., so-called junk DNA. As genomic data can be very large, excluding data from integrity protection can significantly speed up the verification of the data structure. In an embodiment, parts of the multiple genomic blocks are labelled as integrity protected and part of the multiple genomic blocks are labelled as integrity unprotected, only parts labelled as integrity protected being included in the first hash tree.

A hash tree allows the detection of modifications in the data over which the hash tree was computed. Interestingly, a hash tree allows selective verification of data. For example, for partial verification, the hash tree is recomputed insofar it depends on the blocks selected for recomputation, and insofar the hash values in the hash tree were excluded from storage, while using the stored hash values in the hash tree that only depend on data blocks that were not selected.

A hash tree alone, however, does not protect against malicious changes. For example, a malicious change may change data and recompute and replace all hash trees. To avoid this, the encoding system may be configured to compute a digital signature over a root of a hash tree and to include the digital signature in the data structure. Fig. 2a shows an example of this. Block A comprises hash tree information for blocks A1-A3, and also a signature over the hash tree information. To reduce computation, the signature may be computed, not over the entire hash tree information, but only over the root of the hash tree.

In an embodiment, one, or more, or all genomic blocks are stored in compressed form. For example, one, or more, or all of blocks A13-A14 or of block A33-A34 may be compressed. To aid integrity protection, the hash tree may be computed over additional hash values: hash values computed over the uncompressed blocks as well as hash values computed over the compressed blocks.

In an embodiment, one, or more, or all genomic blocks are stored in compressed and encrypted form. Typically, one compresses before encrypting the block. For example, one, or more, or all of blocks A13-A14 or of block A33-A34 may be compressed and then encrypted. To aid integrity protection, the hash tree may be computed over additional hash values: hash values computed over the uncompressed and unencrypted blocks, hash values computed over the compressed and unencrypted blocks, and hash values computed over the compressed and encrypted blocks. These are optional enhancement, an embodiment may only include, say, hash values over the compressed and encrypted blocks, or, say, only over the uncompressed and unencrypted blocks.

Another advantage of hash trees is that if only selected blocks are modified, e.g., amended, the hash tree can be quickly recomputed by using hash values in the hash tree that are computed and stored for blocks that were not amended. If one or more signatures are used, then they may be recomputed as well.

In an embodiment, the device may keep track of such amendments. For example, amendments may be received for the genomic data, e.g., amendments including one or more of additions, deletions, and/or modifications. The hash tree or trees may be wholly or partially recomputed and the tree may be updated in the storage, say. Interestingly, the amendments may be stored as additional blocks. When using the genomic data, the amendments can be applied. The amendments can be included in additional blocks which may be used in a new hash tree or may be included in an existing hash tree. This has the advantage that amendments to the genomic data can be traced.

Amendments to the genomic data may, e.g., comprise amendments to the metadata. Amendments to the genomic data may, e.g., comprise amendments to genomic sequence data. Amendments to the genomic data may, e.g., comprise new data, including new metadata. Amendments to the data structure may be applied and any part of the hash tree that relies thereon, e.g., is computed from amended parts, may be recomputed. In addition, or instead, the amendments themselves may be recorded in the data structure, e.g., to aid accountability. The amendments may be stored in a new data block. A new data block at the leaves level may also avoid that large parts of the hash tree to be recomputed. For example, if before the amendments there were block 1 to 100, then the amendments may be placed in block 101. Advantageously, the amendments are placed in a block at the end so that a large part of the hash tree can be re-used, without recomputation. In this case, a hash over blocks 1 and 2, a hash over blocks 3 and 4, etc. can be re-used. This also works at higher levels, the hash that relies on block 1-4 on the next level, can also be re-used in this example, etc. When reading out the data, the amendments in block 101 may be applied where needed in the data.

The data structure that is constructed in system 200 may be used in various ways.

For example, system 200 may comprise a storage unit 220. Storage unit 220 may be configured to write the data structure to a computer readable medium, e.g., a non-transitory computer readable medium.

For example, system 200 may comprise a streaming unit 230. Streaming unit 230 may be configured to stream the data structure or part thereof over a computer readable medium, e.g., a transitory computer readable medium. For example, streaming may be done over a computer network.

**Fig. 2b** and **Fig. 2c** schematically show an example of an embodiment of a stored encoding. These examples may also be used for streaming, though in streaming one may opt to exclude part of the data structure. For example, in streaming the encoding device may receive a parameter indicated selected part of the data structure. The encoding device may then stream just the selected part, optionally also streaming the blocks that are hierarchically above the selected part. For example, in streaming the encoding device may compute that parts of the hash trees that are not stored, and include them in the streaming. For example, if the sending device is computationally stronger, this may speed up verification at the receiving device.

There are many ways in which a hierarchical data structure can be linearized for storage and/or streaming. For example, the hierarchical relations may be indicated in the blocks with pointers, or labels, or the like. The blocks can be written in various orders. Fig. 2b shows an example, in which blocks are written out depth first. Fig. 2c shows an example, in which blocks are written out breadth first.

In an embodiment, an encoding system is configured for streaming the data structure or part thereof. In streaming the integrity-protected data blocks, a transport message may be included in the stream, e.g., at the start of the stream. The transport message may comprise:

- the overall signature of the one or hierarchy of hash trees, and

    - all hash nodes required for computing the nodes in the path or paths starting from the transported node or nodes to the overall root, so that integrity verification can be performed immediately upon reception of the transported data blocks on the receiving end.

Returning to Fig. 2a. For example, system 200 may comprise a verification unit 240. Verification unit 240 may be configured to verify the data structure or part thereof. For example, verification unit 240 may verify a data structure such as stored or streamed in Fig. 2b or 2c. Verification may entail verification of all the integrity data. For example, all of the parts of the stored hash tree(s) may be recomputed, and the recomputed parts may be compared to the stored parts. Integrity verification may also comprise verification of the signature. Interestingly, verification may also be applied to a selected part of the data structure. For example, the verification unit may recompute multiple hash values for a selected part of the multiple genomic blocks in the data structure, by applying a hash function at least to the genomic data in the selected genomic data block, and recompute the root of the first hash tree from the recomputed multiple hash values the first hash tree for the selected part multiple genomic blocks and from the included levels of the first hash tree for unselected part multiple genomic blocks.

The hash tree may be recomputed insofar it depends on the blocks selected for verification, and insofar the hash values in the hash tree were excluded from storage. For parts of the blocks that were not selected and for which hash values are available, then the hash values from the stored/streamed hash tree may be used.

Verification may take place in a client-server verification system, with the whole data structure stored at the server, and the data blocks to be verified available at the client where verification is performed. For example, in an embodiment, the following verification process may be used; note that the order may be different.

1. Client informs server the ID(s) of the data blocks in the data structure to be verified.
2. Server identifies the path(s) starting from the nodes associated with the data blocks being verified to the overall root of the one or hierarchy of hash trees,
3. Server identifies the data blocks or containers required for computing the nodes in the path(s) identified in the part 2,
4. Server retrieves, if available, or compute the hash nodes associated with the data blocks or containers identified in the part 3,
5. Server sends the retrieved/computed hash nodes computed in the part 4 to the client.
6. Client computes multiple hash values for the data blocks in the data structure selected for data integrity verification, by applying a hash function to the selected data blocks.

7. Client recomputes the overall root of the hierarchy of hash tree(s) from the computed multiple hash values of the data blocks being verified (part 6) and from the computed/retrieved hash nodes received from server (part 5) required for computing the nodes along the path(s) leading to the overall root.

8. Client verifies the recomputed overall root at the top of the one or hierarchy of hash trees in the obtained data structure.

**Fig. 3** schematically shows an example of an embodiment of a hash tree 300. A hash tree such as in Fig. 3 may be used in hash tree computations 211, 212, 213, and so on. The hash tree has multiple levels. A highest level 310 has a single node, the root of the hash tree. A second-highest level 311 contains at least two nodes. In an embodiment, the two nodes of the level 311 are included in storage. Also shown in Fig. 3 is a third-highest level 312 and a leaves level 320, e.g., a lowest level. A selected part of the tree is stored in the data structure. Fig. 3 shows a selected part 330 for storage. Selected part 330 may comprise the first two levels. Selected part 330 may comprise more than the highest two levels. Selected part 330 may comprise part of a level. For example, part of a third-highest level.

In an embodiment, a tree parameter, e.g., a tree size parameter may be used during encoding, e.g., received at the input interface. The tree size parameter may indicate how much of the hash tree is to be stored in the data structure. A tree size parameter may indicate a larger part of the hash tree is to be stored; A tree size parameter may indicate a smaller part of the hash tree is to be stored. In this way, a user can indicate if file size or verification speed for selected parts is to be optimized.

The corresponding verification system, e.g., verification system 160 may be work analogous to encoding system 110 and/or encoding system 200. The verification system may work on the level of an end user, but also at the level of an intermediary, e.g., a server between a source of the data structure and the end user of the data structure. Verification may be done at the server as well as at the end user's device.

For example, a verification system may comprise an input interface configured for receiving at least part of the digital data structure, the data structure comprising multiple genomic blocks and part of a first hash tree, including the first two highest levels of the first hash tree but excluding one or more lower levels of the first hash tree. Note that it is not necessary for the verification system to receive the entire data structure as generated by the encoding device. For example, only part of the genomic data blocks in the data structure may be received, e.g., only the data blocks that are currently of interest. The verification device need not receive all of the hash tree either. For example, parts of the hash tree that rely only on data blocks that are not transmitted and received, can be summarized by sending only the hash value on the highest level of the hash tree that only depends on not received data. For example, if blocks 1-4 are not received, then the hashes of blocks 1-4, not the hashes depending on blocks 1 and 2 or on block 3 and 4, are needed. Assuming block 5 is received, then the hash depending on blocks 1-4 is sufficient information for block 1-4 to verify the signature over the hash tree root. Put in other words, if a particular hash tree node only depends on blocks that are not received, then no hash tree nodes below said particular hash tree node need be transmitted or received, that is no hash tree nodes on which the particular hash tree node depends. Even the particular hash tree node may not be needed if a higher hash tree node is transmitted on which the particular hash tree node depends, and which higher node also only depends on not transmitted data blocks. This optional though, one may send the complete hash tree in so far it is available in the data structure. For example, the received hash tree may include all of a number of higher levels, and omit all of a number of lower levels.

Using the obtained genomic data blocks, e.g., receive genomic data blocks, and the part of the hash tree that is received, the root of the hash tree is recomputed. For those parts of the hash tree that depend on received blocks, the hash values can be recomputed, for hash values that depend on non-received blocks, the received hash value can be used. Recomputed hash tree nodes, in particular the hash tree root can be compared to received hash tree nodes. Furthermore, if there is a signature on the hash tree root, it can be verified as well. If a discrepancy is found in hash values and/or signature, appropriate error handling may be done, e.g., the error may be reported to a user, the file may be rejected, etc.

Instead of verifying all of the received genomic data blocks, the same approach may be used to verify part of the received genomic data blocks.

Below several further optional refinements, details, and embodiments are illustrated. Embodiments below can be applied to enable the long-term integrity protection of genomic files or big data files in general. Embodiments are described in the context of ISO/IEC 23092. ISO/IEC 23092 defines a standard for encoding, compressing, and protecting genomic data. Embodiments advantageous in that context, however embodiments can also be applied outside of that context.

In the current MPEG-G security solution ISO/IEC FDIS 23092-3:2019(E), there are several security issues:

- There is no signature covering the overall MPEG-G file.
- There is no signature to check the overall integrity of a dataset group. In 7.4.2, the rfmd, dgmd (dataset group metadata), and dtpr boxes are protected. However, other boxes, e.g., dtcn, dghd, rfgn, labl and lbll, are not protected.

- A signature can protect a collection of access units that contain the genomic data, or other lower-level data structures that contain the functional data, resolving to bytes of the Value field (defined in ISO/IEC 23092-1:2019, 6.3). However, if it is a collection of access units, this can include up to 2^32 access units, with each of them including up to 256 blocks. This makes it hard to check the validity of selected access units for random access without having to compute on other access units protected in the same collection.
- With data structures being selectively and individually protected by digital signatures, the current solution in general cannot guard against the unauthorized addition, removal or reordering of data structures by an intruder.
- It is not specified the process to verify that a data structure belongs to a given file. It is not specified how to verify that two data structures belong together as part of the same file.

Embodiments address one or more of these issues. Including one or more of the following:

- Problem 1: Current MPEG-G solution applies digital signatures to individual data structures (or containers). But there is no way of efficiently grouping all elements providing a proof of the integrity of the whole file. Preferably, this is achieved with low overhead while keeping random-access.
- Problem 2: Genomic data may be compressed and decompressed, also encrypted and decrypted. Compression algorithms and new software implementations might be prone to software bugs and/or design errors. Thus, there is a need allow verifying the integrity and correctness of encrypted compressed data (ECD), plaintext compressed data (PCD), and plaintext decompressed data (PDD).
- Problem 3: Genomic files are updated. Structures for data file integrity verification preferably enable this need.
- Problem 4: Genomic files are updated. Structures for data file integrity verification preferably enable tracing changes and accountability for those changes.
- Problem 5: Dataset Group ID is very short (8 bits). This makes infeasible to have a unique identifier allowing for some use cases such as merging or retrieval.

Embodiments propose to enhance genomic files, such as MPEG-G files, with a hierarchy of Merkle trees, where each Merkle tree is bound to a data structure in the file, allowing for:

- Long-term integrity protection of all or selected data structures of a file as an integral whole with low storage and computational overhead without hampering random access. This allows for efficient verification of data structures at any level, including at dataset group, and linking all of them together.
- Integrity protection of genomic data while being encrypted and compressed, compressed, and decompressed.
- Traceability and accountability
- Improved performance compared with the current solution

The reason why long-term is highlighted in the first bullet above is that genomic information is relevant for the health of a user and his/her relatives. This means that genomic information preferably remains confidential and private not only during the lifetime of a user, but also of his/her children, and grandchildren. The current MPEG-G solution relies on traditional digital signatures that are not quantum resistant, and which may be broken in the foreseeable time. An option to deal with this problem is to replace the existing digital signatures with quantum-resistant ones, however, quantum-resistant signatures are bulkier and slower than ECDSA. Thus, this will lead to a less efficient solution that requires individual signatures for each data structure under integrity protection. The proposed solution relies on Merkle trees (based on hash functions), and is therefore a natural solution to ensure integrity in the long term with the added benefit that only the root of the tree needs to be signed.

The reason this proposed approach does not hamper random access is that it is possible to access a container and retrieve the required MT nodes to verify that the data in the container has not been modified and the container is part of the overall file without having to access data in the whole file.

While embodiments are described in the context of the MPEG-G standard referencing its specific hierarchy of data structures, most of the features and functionalities are generally applicable to any data formats that organize data into individual components. Embodiments are particularly beneficial for handling large amount of data that are split and stored into a hierarchy of smaller data units.

Below a number of embodiments are described, each building on the previous ones, e.g., Embodiment 2 building on Embodiment 1, Embodiment 3 building on Embodiment 2, etc. Embodiments 1, 2, 3, 4 and 5 address respectively the aforementioned Problems 1, 2, 3, 4 and 5. There are a total of 14 embodiments.

Furthermore, after presenting the embodiments, it is described how embodiments compare with an alternative solution and how embodiments could be applied to GA4GH security solution to protect the file integrity.

### Embodiment 1

This embodiment addresses Problem 1. It associates hierarchical data structures (or containers) in an MPEG-G file to a Merkle tree (MT). In general, containers at higher levels encapsulate data structures at lower levels. Since data structures in an MPEG-G file are organized in a hierarchical way, the roots of the MTs at the lower levels serve as leaves of the MTs at higher levels.

In this embodiment, five levels {1, 2, 3, 4, 5} of Merkle trees are described. The lowest level is 1 and the highest level is 5. At every level, the root of a Merkle tree is obtained by hashing the selected leaf hash codes of the tree concatenated in a predefined order, such as the order in which the leaf data are stored. Hash() denotes a function that generates a hash code of the data structure specified in the brackets.

- Level 1 (MT_AU; MT_DS) : refers to the MTs at Access Unit (AU) and Descriptor Stream (DS) levels in the MPEG-G file.
  ◦ Each access unit forms a Merkle tree whose leaves can include:

    ▪ Hash(AU Header),
    ▪ Hash(AU Information),
    ▪ Hash(AU Protection), and
    ▪ Hash(block) for each data block in the access unit.

In general, the leaves may comprise all or a subset of the elements in AU container defined in Table 25 of ISO/IEC DIS 23092-1.
Table 25 contains:

```
Access_unit {
                Access_unit_header;
                AU_information;
                AU_protection
                If (block_header flag) {
                for (i=0;i<num_blocks;i++) {
                block[i]
                }
                }
                }
```

The root of each access unit MT is denoted as MTR_AU.
◦ Each descriptor stream forms a Merkle tree whose leaves can include:

    ▪ Hash(DS Header),
    ▪ Hash(DS Protection), and
    ▪ Hash(block) for each block in the descriptor stream.

In general, the leaves may comprise all or a subset of the elements in DS container defined in Table 32 of ISO/IEC DIS 23092-1. The root of each descriptor stream MT is denoted as MTR_DS.

- Level 2-a: refers to the MTs at Attribute Group (AG) level in the MPEG-G file.

    ◦ Each attribute group forms a Merkle tree whose leaves can include:

    ▪ Hash(AG Header)
    ▪ One or more MTR_AUs

    In general, the leaves can be all or a subset of the elements in Attribute Group container defined in ISO/IEC DIS 23092-6.
    ◦ The root of each attribute group MT is denoted MTR_AG.
    Level 2-b: refers to the MTs at Annotation Table (AT) level in the MPEG-G file.
    ◦ Each annotation table forms a Merkle tree whose leaves can include:

- Hash(AT Header)
- Hash(AT Metadata)
- Hash(AT Protection)
- One or more MTR AGs

In general, the leaves can be all or a subset of the elements in Annotation Table container defined in ISO/IEC DIS 23092-6.
◦ The root of each annotation table MT is denoted MTR_AT.

- Level 3 (MT_DT): refers to the MTs at Dataset (DT) level in the MPEG-G file.

  ◦ Each dataset forms a Merkel tree whose leaves can include:

    - Hash(Dataset Header)
    - Hash(Dataset Metadata)
    - Hash(Dataset Protection)
    - Hash(Dataset Parameter Set)
    - Hash(Master Index Table)
    - One or more MTR_AUs/MTR_DSs/MTR_ATs

In general, the leaves can be all or a subset of the elements in Dataset container defined in Table 19 of ISO/IEC DIS 23092-1.
◦ The root of each dataset MT is denoted as MTR_DT.

- Level 4 (MT_DSG): refers to the MTs at Dataset Group (DG) level in the MPEG-G file.

  ◦ Each dataset group forms a MT whose leaves can include:

    - Hash(DG Header)
    - Hash(Reference)
    - Hash(Reference Metadata)
    - Hash(Label List)
    - Hash(DG Metadata)
    - Hash(DG Protection)
    - One or more MTR DTs

In general, the leaves can be all or a subset of the elements in dataset group container defined in Table 9 of ISO/IEC DIS 23092-1.

- The root of each dataset group MT is denoted as MTR_DG.

- Level 5 (MT_MPEGG): refers to the MTs at the top of the MPEG-G file that puts together multiple dataset groups.

  ◦ A MT at the file level may comprise leaves that include:

    - Hash(File Header)
    - One or more MTR DGs
  ◦ The root of a MT at the file level is denoted as MTR_MPEGG.
  ◦ At the file level, a new Merkle Tree Integrity Data container (mtid) is introduced for storing MT data used for the verification of file integrity. The mtid box can be placed right after File Header (flhd) or at the end of the file for ease of updating.
  ◦ In its minimal form, the mtid box contains a signature on the overall root MTR_MPEGG and the ID of the signer, who can be the file owner or administrator.
  ◦ The signature is generated with the function Sign(*PrivK, MTR_MPEGG*), where PrivK is the private key of the signer with an associated public key certificate signed by a certificate authority (CA) as a proof of identity.

While in this embodiment five levels of MTs are used, the number of levels is not limited to five. It can be increased to accommodate additional levels of containers introduced into the MPEG-G standard, or reduced for file formats with fewer levels of containers.

Assuming a binary Merkle tree is used to compute the root from the leaves, the way to verify a leaf is described in US patent 4309569, Method of providing digital signatures.

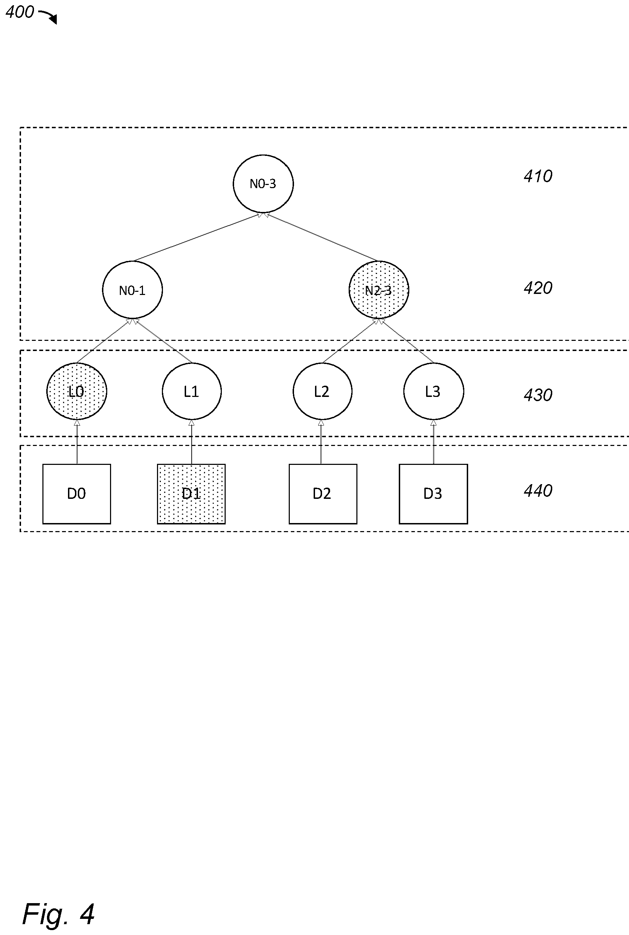

This process is illustrated in the **Fig. 4,** with an example Merkle tree with four leaves: L0, L1, L2 and L3 generated by hashing respectively on the four data elements D0, D1, D2 and D3, which are being protected for overall integrity. The three has a highest level 410, a second-highest level 420, a leaves level 430, and data 440.

For example, D0 may be an AU with and ID, D1 may be metadata, and D2-D3 may be genomic data blocks. Note, to send only the dotted block, in transport mode, the other dotted nodes may be sent as well, to allow recomputation of the root node. The root node, or signature on the root node, N0-3 may also be sent to allow verification of the recomputed root value.

Genomic data is human, animal, or plant genetic information (i.e. information of macromolecules being genes or derived from genes), like a result of sequencing a nucleic acid string, or genederived biopolymers, like a protein.

The tree comprises two internal nodes N0-1 and N2-3, and a root N0-3. To verify the data element D1 for example, the leaf L0 and intermediate node N2-3 are disclosed. Given the data element D1, it is possible to compute L1 as Hash(D1). With L0 and L1, it is possible to compute N0-1 as Hash(L0|L1); with N0-1 and N2-3, it is possible to compute the public root N0-3 as Hash(N0-1|N2-3).

Note that there is a difference between the levels of individual Merkle trees corresponding to the data structures in MPEG-G, and the levels of the internal nodes within a binary Merkle tree. The external Merkle tree levels range from the lowest that is associated with access units or descriptor streams, to the highest that is associated with top-level data containers such as dataset groups. In the case of a binary Merkle tree with $2^n$ data elements, at the lowest level one has $2^n$ leaves generated by hashing independently on $2^n$ data elements, and then one has n levels of intermediate nodes. At each level $l$, where $l = 1, ..., n,$ the number of nodes is given by $2^{(n - l)}$. The root node of the binary Merkle tree is at Level $n.$

When constructing a binary Merkle tree, for an odd number of leaves or nodes at a particular level, one can concatenate the last leaf or node with itself. Consider a five-node example with leaves A, B, C, D, and E. The process is as follows:

- Leaf level: there are 5 leaves, A and B lead to node AB, C and D lead to node CD. E is single, so leads to node EE.

- Level 1: there are 3 nodes, AB and CD lead to node ABCD, EE is alone, so becomes EEEE.

- Level 2: Finally, there are two nodes, ABCD and EEEE, that give the root node ABCDEEEE.

**Embodiment 2:**

This embodiment addresses Problem 2. It comprises in introducing Level 0 in Embodiment 1, where Level 0 is lower than Level 1. This is done as follows:

- Hash(Block) used as a leaf in the Merkle tree for each Access Unit or Descriptor Stream is replaced by the MT root of the block MTR_block computed from (up to) three values for that block:

    ◦ Hash(ECD), the hash of the compressed and encrypted block
    ◦ Hash(PCD), the hash of the compressed block
    ◦ Hash(PDD), the hash of the block after decompressing

    Not all three values are always required. Depending on the values that are included and verified, it is possible to check with more or less accuracy the reason for an integrity failure.

The above hash values may each be assigned to a leaf. This allows to pinpoint an error, e.g., as an error with compression or the like. Note that integrity may be checked at different places. A server cannot check decrypted data, if it has no access to a decryption key, but the server can check the encrypted data.

Furthermore, in an example, the root of a Merkle tree may be

- MTR_Block = Hash(Hash(Hash(ECD) | Hash(PCD)) | Hash(PDD)). Note that in this case a different computation for the root, following e.g., a 3-ary tree structure, is also reasonable, e.g., Hash(Hash(ECD) | Hash(PCD) | Hash(PDD)).
- This allows checking the integrity of the data at different stages of the decryption and decompression process and also at different locations (e.g., client and cloud) in a distributed implementation of the standard.

For instance, assume that an MPEG-G client requests data from an MPEG-G file stored in the cloud. The cloud may check the credentials of the user and use the information in the Merkle tree, namely Hash(ECD) to check that the data encrypted and compressed has not been modified. The cloud may then send to the MPEG-G client the data encrypted and compressed: (i) compressed to save bandwidth, (ii) encrypted since the cloud itself does not have access to the user data. When the MPEG-G client receives the data, it can use Hash(ECD) to check its integrity first. Then, it decrypts the data and can use Hash(PCD) to check the integrity of the decrypted and compressed data. Finally, it decompresses the data and can use Hash(PDD) to check the integrity of the decrypted and decompressed data.

**Embodiment 3:**

This embodiment addresses Problem 3. To this end, when data is added and/or modified in a data structure at Level 0, 1, 2, 3 or 4, then:

- A new/updated MT is computed for that new data structure at Level i
- The following process is executed until MTR_MPEGG, and accordingly the signature on MTR_MPEGG, are updated at level 5.

  ◦ A new (or updated) leaf is to be added to the next upper-level MT. The value of the new (or updated) leaf is the value of the MTR of the MT at the current Level i.
  ◦ Since the MT at the next upper level has a new/updated leaf, its root is updated as well.

**Embodiment 4:**

This embodiment addresses Problem 4. To this end, one option is to include a tracking table is included in the mtid box described in Embodiment 1 to trace changes/updates (**) in the MPEG-G file. The $i^{th}$ entry in the table that corresponds to the $i^{th}$ generated signature since the creation of the file, with i = 0, 1, 2, ..., contains:

- All or selected root values MTR_DG, MTR_DT, MTR_AT, MTR_AG, MTR_AU, MTR_DS and Merkle Tree nodes to allow for efficient verification and storage. For more details, see Embodiments 6-8.
- A signature on the current MTR_MPEGG denoted SIGNATURE_MTR_MPEGG(i) computed as Sign(PrivK, i | MTR_MPEGG| SIGNATURE_MTR_MPEGG(i-1)), where

  ◦ SIGNATURE_MTR_MPEGG(-1) = ""

  ◦ PrivK is the private key of the signer with an associated public key certificate signed by a certificate authority (CA) as a proof of identity.

  ◦ When aiming at long-term integrity protection, a hash-based signature algorithm such as LMS, see, e.g., RFC8554 or one of the quantum resistant algorithms, e.g., Falcon, SPHINCS (currently under standardization by NIST) is preferred. In particular, if a pre-quantum signing algorithm is used (e.g., ECDSA) in some entries, those entries may not be able to be verified as soon as, in this case, ECDSA is broken, e.g., due to quantum computers.

- ID of the signer

When a file is modified at time i, with i = 1, 2, ...,

- MTR_MPEGG is to be updated as described in Embodiment 3.
- A new entry may be included in the tracking table in mtid. To facilitate tracing, the tracking data can include:

  ◦ SIGNATURE_MTR_MPEGG(i)
  ◦ The new added data (in case of data addition) or the diff of the modified data compared with the previous data.
  ◦ The leaves that have been modified or added.

Furthermore,

- When one accesses the data of a leaf, by going through the path to the current Merkle tree root, one can check the file integrity and all related modifications for that piece of data.
- When one searches for a specific data item in the tracking table in mtid, one finds all information for the addition/modification of that piece of data.

**Embodiment 5:**

This embodiment addresses Problem 5. This may be done by:

- Making datasetGroupID long enough (e.g., 256 bits long) and generating it at random.
- In particular, replacing datasetGroupID with MTR_DG. To ensure that datasetGroupID is unique, an additional input in the computation of MTR DG is included, namely a long enough random nonce N_MTR_DG.
- Similarly, datasetID can be replaced with MTR_DS. To ensure that datasetGroupID is unique, an additional input in the computation of MTR DG is included, namely a long enough random nonce N_MTR_DG.

An advantage of this change is that each dataset or dataset group is assigned a unique identifier. This is an advantage since the current MPEG-G standard includes use cases for merging files and an API to retrieve entries based on these identifiers. If the identifier range is as small as 8 bits, merging two dataset groups may require renaming the identifiers. If the identifiers are not renamed, then a call to the API can return the wrong result. Using MTR_DG and MTR_DS as datasetGroupID and datasetID solves these two problems.

**Embodiment 6: MT Storage Overhead and Computational Performance Trade-offs and Optimizations:**

In general, for data structures that exist in small numbers, the whole Merkle Tree (MT) can be stored for improving the efficiency of integrity validation without incurring much storage overhead. On the other hand, for data structures that usually exist in large numbers, a trade-off between storage overhead and computational resources is preferred. For a binary MT with $2^n$ leaves, the computation of the MT nodes and root takes ($2^n - 1$) hash operations, if all $2^n$ leaves are available. There are a total number of $2^n - 1$ nodes in the binary MT, including all intermediate nodes and the root.

This embodiment proposes as optimization to store the top $m$ levels of nodes, $1 \leq m \leq n$, counting from the root, of a binary MT. If this set of nodes together with all the leaves are stored, the computational burden to verify a leaf is reduced to $[2^{(n - m + 1)} - 1 + (m - 1)]$ hash operations on pairs of nodes. The storage of leaves avoids the expensive operation of hashing on the data structures, in particular, if they are large.

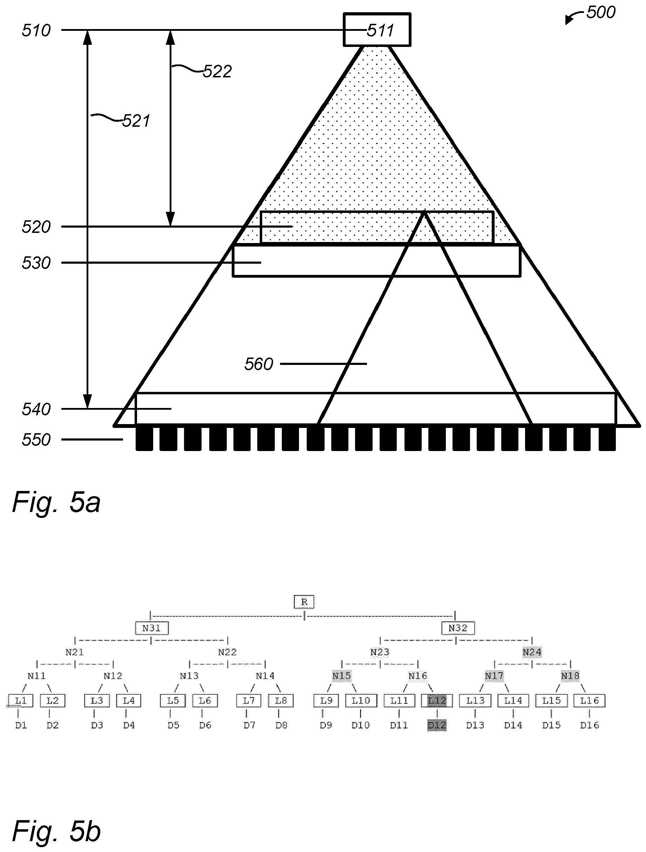

This optimization is shown in the **Fig. 5a** where the dotted triangle represents a partial Merkle tree. Shown in Fig. 5a, is a root node 511 at the highest level 510, say at level $n$. Also indicated is level $n - (m - 1)$ at 520 and level $n - m$ at 530, and level 1 at 540 and a leaves level (level 0) at 550. There are $m$ levels indicated at 522, and $n$ levels at 521. Level 520 includes $2^{n-(m-1)}$ nodes. Level 530 includes $2^{n-m}$ nodes. The doted triangle part includes $2^m - 1$ nodes. Level 1 has $2^{n-1}$ nodes. Level 0 has $2^n$ nodes.

Triangle 560 indicates nodes that may be generated on the fly, when needed. Triangle 560 comprises $2^{n-m+1} - 1$ nodes. The undotted part of tree 500, e.g., levels 530 and below, are not stored. The top levels, e.g., level 520 and above are stored.

The whole Merkle tree in this example has $2^n$ leaves. Both the leaves and the nodes in the top $m$ levels, including the root, are stored. The triangle 560 in the bottom ($n - m + 1$) levels are recomputed on the fly when a specific leaf needs to be verified.

There can be a large number of leaves, e.g., as much as, $2^{32}$. There could be even more leaves if required. Block size can be varied as well. A smaller block size allows for finer grained integrity checking with little overhead, though the size of the hast tree may increase. For example, a tree size parameter may be used to determine block size.

The part of the MT that is stored can be part of the file. Can be stored as a sperate file. This can be stored as part of the data structure.

For instance, if $n = 32$, $m = 24$ and assuming a hash code size of 32 bytes, it stores ($2^{24} - 1$) hashes (~$2^{24}$ * 32 bytes) for the top part of the tree and the $2^{32}$ leaves. Verifying a leaf takes ($2^9 - 1$) hash operations for the recomputation of the partial MT at the bottom, plus 23 hashes to reach the root. ($2^9 + 22$) operations is very fast. The memory overhead heavily depends on the number of leaves of the data structure.

As an alternative, if the $2^n$ leaves are not stored, just by storing the top $m$ levels of nodes can still reduce the number of leaves that need to be computed for integrity validation to $2^{(n - m + 1)}$. In this case, the storage overhead is given by ($2^m - 1$) hashes, and the time for recomputing the Merkle tree root is given by:

$$T_{binary\_MT} = [2^\wedge(n - m + 1) - 1 + (m - 1)] * t_{node} + 2^\wedge(n - m + 1) * t_{leaf}$$

where $t_{node}$ is the time for hashing on two nodes and $t_{leaf}$ is the time for generating a leaf by hashing on a data structure.

Without the storage of any leaves or nodes, the computational time becomes

$$T_{binary\_MT} = [2^\wedge n - 1] * t_{node} + 2^\wedge n * t_{leaf}$$

The operation of a binary MT in a client-server setting, where the file resides at the server and verification takes place at the client, is further explained using the example shown in Fig. 5b with $n = 4$ and $m = 2$. Fig. 5b shows a hash tree with levels 4-1 and a leaves level. Level 4 has node R; level 3 has nodes N31 and N32; Level 2 has nodes N21, N22, N23 and N24; Level 1 has nodes N11-N18; Leave level has nodes L1-L16 corresponding to data D1-D16.

Suppose all nodes in Levels 3 and 4, and all the leaves are pre-computed and stored, e.g., in a data block. In order to validate D12 (highlighted), the following steps may be taken:

1. Server generates the nodes {N15, N17, N18, N24} (highlighted). In general, the number of hash operations at the server is given by {[2^(n - m + 1) - 1] - (n - m + 1)}.

2. Server sends the hashes {L11, N15, N24, N31} together with the signature of the root to the client. In general, the number of hashes that need to be sent is $n$.

3. Client generates:

- Leaf L12 by hashing on the data component D12

- Nodes {N16, N23, N32, R} (highlighted) using the hashes received from the server. In general, the number of hash operations on pairs of hashes at the client is $n$.

4. Client decrypts the received signature using the associated public key and compares the decrypted hash code with the generated root R. The validation is successful if they match with each other.

Compare the cost of this approach with the current MPEG-G, which requires one digital signature per data structure being protected for integrity, this approach has:

- Lower computational overhead as soon as two data structures need to be verified. The reason is that the cost of digital signature verification is much more expensive than a hash computation. The MT approach involves a single signature verification for the MT root of the file.

- Lower storage overhead, assuming that the same data structures are integrity protected. The reason is that the current MPEG-G requires a digital signature per data structure, and the signature size is bigger than a hash value, in particular, if a quantum resistant signature is used.

The following table summarizes the properties of different levels of Merkle trees including the tree name, the maximum number of leaves, the storage need, the number of hash operations to verify a leaf, and the number of internal levels in the binary tree.

| Level | Data Structure Description | Tree Name | Max. Num. of Leaves | Storage (Num. of Hash Codes) | Num. of Hashings for Validation | Max. Num. of Levels in the Binary Tree |
|---|---|---|---|---|---|---|
| 0 | The size of a data block is specified by a 32-bit unsigned integer, e.g., u (32). This means it can be up to 4 GB. | MT_Block (Embodiment 2) | 3 | 3 (root and 1st level) + 3 (leaves) | 2 | 2 |

(continued)

| Level | Data Structure Description | Tree Name | Max. Num. of Leaves | Storage (Num. of Hash Codes) | Num. of Hashings for Validation | Max. Num. of Levels in the Binary Tree |
|---|---|---|---|---|---|---|
| 1 | The number of blocks in an Access Unit (AU) is in data type u(8), with a maximum of 256. | MT_AU | 256 | 2^8 - 1 (all MT nodes) + 256 (leaves) | 8 | 8 |
| 1 | The number of blocks in a Descriptor Stream (DS) is in data type u(32), with a maximum of 4,295 M. | MT_DS | 2^32 | 2^24 - 1 (top 24 levels incl. root) + up to 2^32 (leaves) | 2^8 + 24 - 1 | 32 |
| 3 | The number of AUs in a dataset is in data type u(32), with a maximum of 4,295 M. | MT_DT | 2^32 | 2^24 - 1 (top 24 levels incl. root) + up to 2^32 (leaves) | 2^8 + 24 - 1 | 32 |
| 3 | The number of DSs in a dataset is in data type u(7), with a maximum of 128. | MT_DT | 128 | 2^7 - 1 (all MT nodes) + 128 (leaves) | 7 | 7 |
| 4 | The number of datasets in a dataset group is in data type u(16), with a maximum of 65,536. | MT_DSG | 65,536 | 2^16 - 1 (all MT nodes) + 2^16 (leaves) | 16 | 16 |

## Embodiment 7: Use of Authentication Tag in Place of Hash Code for an Encrypted Data Container

Note that MPEG-G allows protecting the different containers with a symmetric key by using AES in GCM mode. This means that a data container can be encrypted and also authenticated by using the corresponding symmetric key. Authentication is performed by checking that the stored authentication tag that depends on the whole data container. The authentication tag in the existing MPEG-G solution could be reused as the fingerprint of the data container in order to reduce CPU and memory requirements. In particular, the AES-CCM authentication tag could replace, e.g., hash(block) in Level 1 of Embodiment 1 or hash(ECD) in Embodiment 2. If the authentication tag is reused as a fingerprint, then the hash of the data container (a MT leaf) does not need to be recomputed or stored.

## Embodiment 8: Selective Integrity Protection

In an embodiment, all data structures, or containers are protected. However, in some circumstances a user might opt for partial protection. This embodiment describes how the selective protection of data structures can be realized.
To this end, each container type may have a field to indicate how the data structures of the container type are selected in general. For example, the value of the field could be:

- 0 - none is selected (the Merkle Tree excludes all data structures at this container level and below)
- 1 - all are selected
- 2 - select data structures by ID
- 3 - select data structures by flags, e.g., using a sequence of bits to indicate the selection of data structures according to their positions in the upper-level container

The following are example settings for the general selection modes of the Dataset Group, Dataset, Annotation Table, Access Unit and Block containers for their inclusion in MT validation:

- mt_select_dg = 1 (select all dataset groups)
- mt_select_dt = 2 (select datasets by ID)
- mt_select_at = 2 (select annotation tables by ID)

- mt_select_au = 2 (select access units by ID)
- mt_select_bl = 1 (select all payload blocks)

These general selection settings for each container type can be overridden for individual containers. Note that once a container is excluded, all its subordinate data structures are excluded regardless of their selection settings.

### Embodiment 9: Selective Storage of Hash Codes for Improving Speed of Integrity Validation

Storage of hash codes can improve the speed for signature validation at the cost of storage overhead. As described in Embodiment 6, the storage cost is lighter towards container levels closer to the root (fewer number of hash codes) and with more benefits for improving computational speed (each hash code representing a larger chunk of data). This embodiment describes how the MT hash codes (or nodes) can be selectively stored.

To this end, each container type may have a field to indicate how its corresponding MT hash codes, including both leaves and other nodes in the binary tree (refer to Embodiment 6), are to be selected for storage in general. For example, the value of the field could be:

- 0 - none of the nodes
- 1 - all nodes in the binary MT, including all leaves and intermediate nodes
- 2 - all leaves only
- 3 - nodes in the top $m$ levels of the binary MT
- 4 - first $n$ number of leaves
- 5 - all leaves plus nodes in the top $m$ levels of the binary MT
- 6 - first $n$ number of leaves plus nodes in the top $m$ levels of the binary MT

The following are example settings for the general storage modes of the MTs at the File (MPEGG), Dataset Group (DG), Dataset (DT), Annotation Table (AT) and Access Unit (AU) levels:

- mt_store_mpegg = 1 (store all MT nodes at the overall file level, with leaves being MTR_DGs)
- mt_store_dg = 1 (store all MT nodes at Dataset Group level, with leaves being MTR_DTs)
- mt_store_dt = 1 (store all MT nodes at Dataset level, with leaves being MTR_ATs)
- mt_store_at = 1 (store all MT nodes at Annotation Table level, with leaves being MTR_AUs)
- mt_store_au = 0 (no MT nodes at Access Unit level are stored)

For storage modes 3-6, additional field(s) are needed for specifying the number of top levels ($m$) and the number of leaves ($n$) in the binary MT to be stored. These general MT storage settings for each container type can be overridden for individual containers.

### Embodiment 10: Full or Targeted Integrity Validation

With some or all of the hash codes on the Merkle Tree being stored, to validate the integrity of a particular component, it is only needed to regenerate the hash code that covers the component in question. Then, all the hash codes tracing back from the component in question to the root of the tree are validated and finally the signature is checked.

### Embodiment 11: Flexible Merkle Tree Organization - Binary vs K-arv Trees. Single vs Multiple Tree Hierarchies

The description in above embodiment focuses on a hierarchy of Merkle trees organized as in the MPEG-G file data structures. Each protected data structure is associated with an independent Merkle tree. Embodiments typically assume binary Merkle trees, e.g., each node in the tree has at most two children. So a data file, such as a MPEF-G file may have a hierarchy of binary Merkle trees, each corresponding to a data container. As an alternative to the binary tree configuration, a K-ary Merkle tree comprises nodes each computed by hashing on k subordinate nodes at a time. In the extreme case, $k$ can be equal to the total number of leaves, resulting in a tree depth of one. In other words, the root is computed by directly hashing on a concatenation of all $N$ leaves. Assuming the hash function has a linear time complexity of $O(N)$ and $N = 2^n$ for ease of comparison with the binary model, the time for recomputing the root of the N-ary Merkle tree is given by:

$$T_{N\text{-}ary\_MT} = 2^{(n-1)} * t_{node} + 2^n * t_{leaf}$$

where $t_{node}$ is the time for hashing on two nodes and $t_{leaf}$ is the time for generating a leaf by hashing on a data structure.

As described in Embodiment 6, the time for recomputing the root of a binary Merkle tree with the top $m$ levels of nodes stored is given by:

$$T_{binary\_MT} = [2^{\wedge}(n - m + 1) - 1 + (m - 1)] * t_{node} + 2^{\wedge}(n - m + 1) * t_{leaf}$$

Comparing the two approaches, with a storage overhead of $(2^{\wedge}m - 1)$ hash codes, where $1 \leq m \leq n$, assuming: (1) $t_{leaf} \gg t_{node}$, (2) the number of leaves is relatively small, e.g., in the case of MT_AU, where the maximum number of leaves is 256, and (3) no mixed storage of nodes and leaves for the binary Merkle tree approach:

$$T_{N\text{-}ary\_MT} \approx (2^{\wedge}n - 2^{\wedge}m + 1) * t_{leaf}$$

$$T_{binary\_MT} \approx 2^{\wedge}(n - m + 1) * t_{leaf}$$

When $m = n$, $T_{N\text{-}ary\_MT} \approx t_{leaf}$ whereas $T_{binary\_MT} \approx 2 * t_{leaf}$.

*When $m = n - l$, where $1 \leq l \leq (n - 1)$,*

$$T_{binary\_MT} \approx 2^{\wedge}(n - m + 1) * t_{leaf} = 2^{\wedge}(l + 1) * t_{leaf}$$

$$T_{N\text{-}ary\_MT} \approx [2^{\wedge}n - 2^{\wedge}(n - l) + 1] * t_{leaf} > 2^{\wedge}(l + 1) * t_{leaf} * \{2^{\wedge}[n - (l + 1)] - 2^{\wedge}[n - 2(l + 1)]\}$$

$$> 2^{\wedge}(l + 1) * t_{leaf} \approx T_{binary\_MT}$$

Based on above calculations regarding the speed of integrity validation, under the assumption of negligible computational time of hashing on the nodes compared with generating the leaves, it can be concluded that:

- The binary MT approach is faster when the storage overhead is $\leq (2^{\wedge}n - 4)$ hashes. The reason is that with $m = (n - 1)$ or a storage overhead of $[2^{\wedge}(n - 1) - 1]$, $T_{binary\_MT} \approx 4\ t_{leaf}$. This computational time remains the same until the storage overhead becomes large enough to cover the next level, e.g., $m = n$ or a storage overhead of $(2^{\wedge}n - 1)$. Whereas for the N-ary approach, when the storage overhead is $\leq (2^{\wedge}n - 4)$ hashes, $T_{N\text{-}ary\_MT} \geq 4\ t_{leaf}$.
- The N-ary tree approach is faster when the storage overhead is between $(2^{\wedge}n - 3)$ and $2^{\wedge}n$ hashes.
- In the binary MT approach, when the storage overhead is $> 2^{\wedge}n$ hashes, the $2^{\wedge}n$ leaves can first be stored and the rest of the overhead can be used to store the internal MT nodes, leading to further improvement in validation speed.

Considering the computational time of hashing on the nodes only, the binary approach always outperforms the N-ary approach when $m > 1$ since

$$T_{binary\_MT\_nodes\_only} = [2^{\wedge}(n - m + 1) - 1 + (m - 1)] * t_{node} < 2^{\wedge}(n - 1) * t_{node} < T_{N\text{-}ary\_MT\_nodes\ only}$$

Therefore, overall the binary MT approach has a better performance than the N-ary approach, except in a narrow range of storage overhead from $(2^{\wedge}n - 3)$ to $(2^{\wedge}n + 2)$ hashes, which correspond to the cases of storing only the top $m \geq (n - 1)$ levels of nodes, or storing all the leaves plus the root ($m = 1$). Under such a specific storage overhead constraint, there is no clear winner in validation speed.

These analysis results can provide guidelines for the choice of binary or N-ary Merkle tree approaches based on the number of selected leaves (Embodiment 8) and the storage mode settings (Embodiment 9). Note that in a client-server setting, where the file resides at the server and verification takes place at the client, the binary approach sends only $n$ nodes to the receiver, whereas the N-ary approach requires $(2^{\wedge}n - 1)$ leaf nodes to be sent.

There could also be an advantage for allowing more flexible Merkle Tree organizations. One flexibility is to allow having multiple Merkle trees per file, e.g., MT_1, MT_2, ..., each comprising data belonging to the same cohort with integrity to be protected as an integral whole independently, or each with a different set of parameters for specific integrity protection requirements. It is also possible to support nested Merkle trees, e.g., by merging k Merkle trees into one big tree with the root of the overall MT computed as the hash of the roots of the k Merkle trees a level deeper: MTR_Overall = Hash(MTR_1 | MTR_2 | ...| MTR_k).

**Embodiment 12: Splitting into Merkle Trees for Functional and Data Components**

An alternative embodiment related to Embodiment 10 is to organize the functional and data components into separate Merkle tree hierarchies, which are then joined together to form a new overall root. Functional components include the header, metadata, and protection structures at different container levels, whereas data components correspond to structures containing the payload block data. Since the functional components are generally important and small in size, it seems reasonable to impose that all functional components be protected by a Merkle tree without selection, while allowing the selective protection of the data components. One potential advantage of such arrangement is faster validation of the functional components. For example, to validate the metadata of a dataset, the hash codes of individual access units in the dataset are not involved in the validation. Only the hash code at the root of the Merkle tree for the data components is used for the validation. The following example illustrates the idea of splitting the functional and data components into two separate Merkle trees and then joining them into a single overall root:

MTR_MPEGG

- MTR_Functional

    - MTR_Functional_DG_1 = Hash(Header_DG_1 | Metadata_DG_1 | Protection DG_1 | MT_Functional_DT_11 | MT_Functional_DT_12 | ...)
    - MTR_Functional_DG_2 = Hash(Header_DG_2 | Metadata_DG_2 | Protection_DG_2 | MT_Functional_DT_21 | MT_Functional_DT_22 | ...)

- MTR_Data

    - MTR_Data_DG_1
    - MTR_Data_DG_2

In this example, the overall root MTR_MPEGG depends on the roots of the functional and data Merkle trees as Hash(MTR_Functional | MTR Data). MTR_Functional is obtained by hashing on a concatenation of functional MT roots at the Dataset Group level. Such roots, prefixed by MTR_Functional_DG, are generated by hashing on a concatenation of Dataset Group Header, Metadata, Protection, and subordinate functional MT roots at the Dataset level. Since in generating a root value, the hash function takes data components at the same level as multiple inputs, this resembles a K-ary Merkle tree.

**Embodiment 13: Timestamps**

A timestamp can be included in each Merkle tree data structure to indicate the freshness of integrity protection by the Merkle tree. The timestamp may be stored and signed along with the root of the top Merkle tree, MTR_MPEGG. Furthermore, a freshness period can be imposed such that a signature on the overall MT root is automatically regenerated with an updated timestamp before the expiration of the MT data. An expired timestamp indicates that the file may not be up-to-date, and could be an older version used by an intruder to masquerade as current version and undo any latest changes.

An alternative is that when some data structures are featured by specific dates, the date is used as an input (e.g., as additional leave) in those data structures. For instance, assume that a user has carried out some analyses on the genomic data of a patient during several days. If the analyses are reflected in multiple data structures that have been modified at different instants of time (different hour, different day), each of the data structures can have a different timestamp when building the new overall hierarchy of Merkle trees. The user may include a timestamp when signing the MTR_MPEGG. The timestamp of the signature is preferably the latest timestamp.

**Embodiment 14: Usage for Transport Mode**

MPEG-G defines options for storage and transport of data. In an embodiment, when transporting data, messages can be integrity protected as well.

When a data structure, e.g., block of data, is sent, the message may start with:

• the nodes in the Merkle trees required to verify the integrity of the data structure / block of data, and

- the file signature.

This allows verifying the integrity of the block of data and the block of data being a part of the file without having to receive the whole file first.

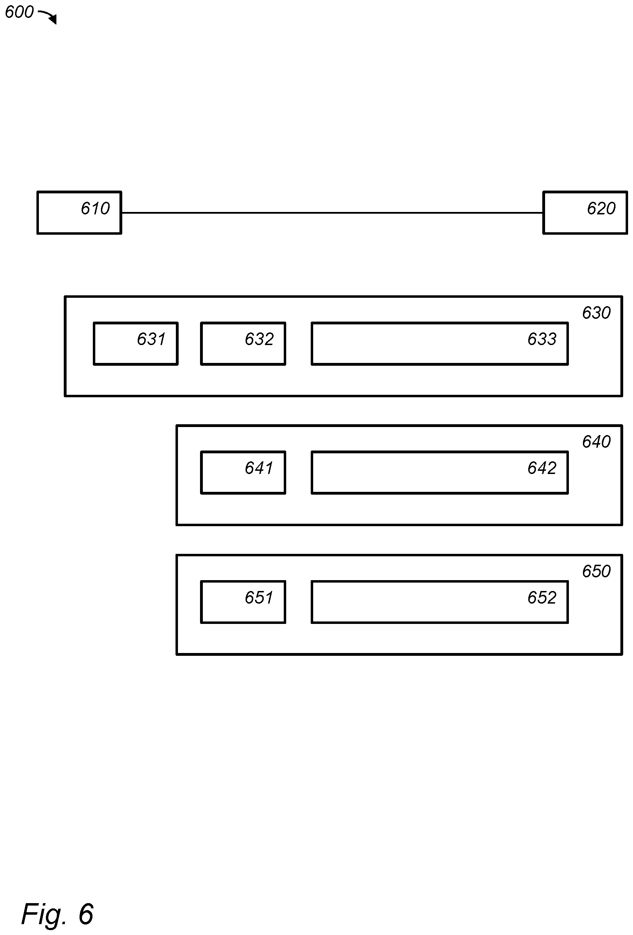

The message can include the signature on MTR_MPEGG, e.g., if this is the first message. Later messages do not need to include this signature again, since this would be redundant. This signature also only needs to be verified for the first message. This is illustrated in Fig. 6 in which n blocks of data are retrieved by a client from the server. This n blocks of data are exchanged in n messages. The first message may include: the file signature, the public key of the signer (if not already available at the receiver), the nodes involved in the Merkle tree path required for verifying the first block of data, and the block of data itself. When the client receives this information, the client may compute the hash on the received data (block 1), computes the root of the tree using the generated hash and the required nodes in the path, and verifies the signature using the generated root and the public key of the signer.

Shown in Fig. 6 is a client 610 and a server 620. A message 1 (630) comprises a signature (631), the nodes involved in the Merkle tree path required for verifying block 1 (631), and a block 1 (633). A message 2 (640) comprises, the nodes involved in the Merkle tree path required for verifying block 2 (641), and a block 2 (642). A message 3 (650) comprises, the nodes involved in the Merkle tree path required for verifying block 3 (651), and a block 3 (652).

The processing of later received messages is similar with the difference that the signature does not need to be included/checked anymore since it is unique for the whole file.

**Comparison with an Alternative Solution Based**

Embodiment above use a Merkle tree in their data structure to achieve long-term integrity protection of MPEG-G files. An alternative solution comprises modifying/extending the current MPEG-G solution in 23092-3:2019-3, 7.4.

For example, in the alternative solution may comprise two changes: Use of a long-term signature algorithm and defining a procedure that allows linking signed containers together

The first point can be addressed by using a quantum-resistant algorithm, e.g., a hash-based signature algorithm such as LMS, XMSS or SPHINCS instead of ECDSA.

As for the second point, different approaches can be considered. Below, three options are described:

1. A first approach comprises including a unique file identifier, e.g., a randomly generated 256-bit long identifier, in the file header. This file header is signed by the system administrator. To bind together all data containers in the file, the MPEG-G specification may be updated in such a way that when a container is signed, then this signed container may include the unique file identifier. If this is done, then when a user retrieves a data container from a file, the user needs to:

- Retrieve and verify the signature main file header and extract the file identifier ID_file.
- Retrieve the data container, verify its signature, and check that the ID in the data container is equal to ID file.

2. The previous approach is that data in different MPEG G files might be merged. When two files are merged, a new file header may be created including a new file identifier. The file header may include the merging history including the identifiers of the previous files. If this is done, then when a user retrieves a data container from a file, the user needs to:

- Retrieve and verify the signature main file header and extract the file identifier IDfile, as well as previous file identifiers from previous unmerged files.
- Retrieve the data container, verify its signature, and check that the ID in the data container is equal to IDfile, or one of the IDfiles from previous unmerged files.

3. Another alternative comprises in each container having a unique long identifier (e.g., a 256-bit long identifier generated randomly). When a container is signed, the signature may include the identifier of the parent container. If the signature of the container is to be verified, the following process is followed:

- Check the signature of the container and retrieve the ID of parent container
- Repeat until ID of parent container corresponds to the MPEG-G header file

  - Check signature
  - Retrieve ID of parent container

4. Another option is to use the protection box specified in MPEG-G Part 3 in Section 7.4 and define a process linking them together. For instance, when a block of data in an access unit is to be verified, one or more or all of the following steps may be carried out:

> 1. A digital signature must be present in the access unit protection for given block;
> 2. The verification of this first signature must be successful.
> 3. There must a signature of the access unit protection for the given access unit at the dataset level in the dataset protection box.
> 4. The verification of this second signature must be successful.
> 5. There must be a signature of the dataset protection box for the given dataset at the dataset group level in the dataset group protection box.
> 6. The verification of this third signature must be successful.

If any of the steps fails, the integrity verification process fails. If a step is successful, then the next step is evaluated.

Even in the case that all the above steps are individually performed, this verification process that links them together is missing in the current MPEG-G specification. Furthermore, there is no definition of a signature on the overall file linking all dataset groups together.

Compare the above methods with embodiments using Merkle trees, the embodiments using Merkle trees is more efficient and comprehensive. There are several reasons:

> a) The MT-based solution allows verifying not only that the integrity of individual components, but also that they belong together as part of the overall MPEG-G file. It prevents any unauthorized addition, removal or reordering of data structures in a file.
> b) The first reason for this is that the MPEG-G approach requires storing a signature per container and hash-based signatures (e.g., SPHINCS) are big. See, e.g., the paper "SPHINCS: practical stateless hash-based signatures", by Bernstein, et al.
> c) Since the hash-based signatures for each container need to be independently validated, the computational overhead is much larger.

## Application to Ensure Integrity Protection in GA4GH files

The Global Alliance for Genomics and Health (GA4GH) describes in its paper ("GA4GH File Encryption Standard", 21 October 2019) how to encrypt and integrity protect individual blocks of 64 kilobytes that are then exchanged. Each of the blocks is encrypted and a message authentication code (MAC) is added. However, the solution does not prevent an attacker from inserting, removing, or reordering entire blocks (this is stated in Crypt4GH, Section 1.1) during communication.

An approach to deal with this problem comprises in defining a Merkle tree in which each of those blocks of 64 kilobytes is a leaf. This is equivalent to Level 1 in Embodiment 1. The blocks of data could also be individual small Merkle trees as in Embodiment 2.

The root of the Merkle tree could be signed, or alternatively, a MAC can be computed using the same key and MAC algorithm as in Crypt4GH.

Assuming there are n blocks of 64 kilobytes. When a block of data is to be transmitted, then the following information is to be sent:

> a) the signature (or MAC) on the Merkle tree root, and
> b) the corresponding log(n) nodes in the binary Merkle tree that allow checking the block of data (this includes the block index and node IDs of the Merkle tree),
> c) the encrypted and MAC protected block of data (according to Crypt4gh, up to 64 kilobytes in size).

Note that if the block size is chosen to be B = 64 kilobytes due to potential fragmentation issues when transporting the data, then the additional integrity data in points a) and b) above may be considered.

This means that the total size of the block can be up to B = 64 kilobytes and the data block may be somewhat smaller due to the overhead caused by a) and b).

When receiving a message, the receiving party uses the received log(n) nodes in the Merkle tree to recompute the root of the Merkle tree and identify the position of the block of data. Then, the receiving party checks the signature on the Merkle tree. Finally, the receiving party checks the received block of data. Note that the root signature only needs to be verified for the first message. This is as in Embodiment 14.

Observe that the current specification of Crypt4GH - despite the statement in Crypt4GH, Section 1.1 - might also offer some integrity protection, e.g., to prevent unauthorized insertion, removal or reordering of data blocks, if an index is included in the block of data. This feature is of independent interest. This index identifies the relative position of the blocks. If this index is included in each exchange block in Crypt4GH, then it is not possible to reorder the blocks anymore. The receiver can also check that there are no duplicates based on this index. If a receiver has received block with index $k$, then the receiver might also check whether it has received all blocks with block indices 1, 2, ..., $(k - 1)$.

This last "index-based" approach is different from the MT-based approach. An advantage is that while the "index-based" approach is about how the data is sent, the MT approach also gives information about how the data is stored. This means if an attacker can influence the process of sending packets, the attacker might place the indices in the right order but swap the blocks. The receiving party may then assemble the file in the wrong order. With the MT based approach, this is not feasible, since the MT gives information about how the blocks are organized and stored in a file.

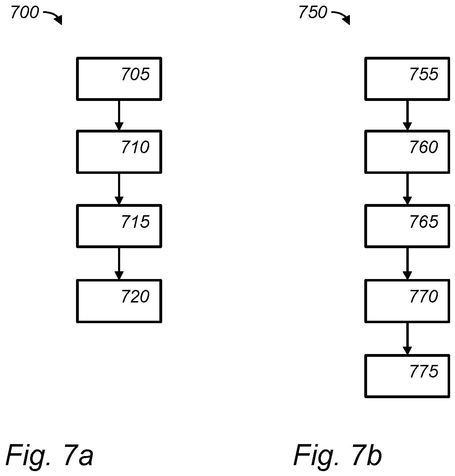

**Fig. 7a** schematically shows an example of an embodiment of an encoding method 700 for encoding data in a digital data structure. The encoding method comprises

- obtaining (705) the data as multiple data blocks,
- computing (710) multiple hash values for the multiple data blocks, by applying a hash function to the multiple data blocks,
- computing (715) a first hash tree for the multiple hash values, the multiple hash values being assigned to leaves of the first hash tree, one or more higher levels of the first hash tree being generated,
- including (720) in the data structure the multiple data blocks and part of the first hash tree, said part excluding at least part of the leaves of the first hash tree.

Obtaining the multiple genomic data blocks may comprise receiving the genomic data blocks directly.

**Fig. 7b** schematically shows an example of an embodiment of a verification method 750 for verifying selected genomic data in a digital data structure. The verification method 750 comprises

- receiving (755) at least part of the data structure, the data structure comprising multiple data blocks and part of a hash tree, excluding at least part of the leaves of the first hash tree,
- computing (760) multiple hash values for data blocks in the data structure selected for data integrity verification, by applying a hash function to the selected data blocks,
- identifying (765) a path starting from data blocks selected for verification to a root of a corresponding hash tree,
- retrieving (770), if available in the data structure, or compute the hash values for the hash tree along the path,
- verifying (775) the root of the hash tree from at least the computed multiple hash values.

Many different ways of executing the method are possible, as will be apparent to a person skilled in the art. For example, the order of the steps can be performed in the shown order, but the order of the steps can be varied or some steps may be executed in parallel. Moreover, in between steps other method steps may be inserted. The inserted steps may represent refinements of the method such as described herein, or may be unrelated to the method. For example, some steps may be executed, at least partially, in parallel. Moreover, a given step may not have finished completely before a next step is started.

Embodiments of the method may be executed using software, which comprises instructions for causing a processor system to perform method 700 and/or 750. Software may only include those steps taken by a particular sub-entity of the system. The software may be stored in a suitable storage medium, such as a hard disk, a floppy, a memory, an optical disc, etc. The software may be sent as a signal along a wire, or wireless, or using a data network, e.g., the Internet. The software may be made available for download and/or for remote usage on a server. Embodiments of the method may be executed using a bitstream arranged to configure programmable logic, e.g., a field-programmable gate array (FPGA), to perform the method.

It will be appreciated that the presently disclosed subject matter also extends to computer programs, particularly computer programs on or in a carrier, adapted for putting the presently disclosed subject matter into practice. The program may be in the form of source code, object code, a code intermediate source, and object code such as partially compiled form, or in any other form suitable for use in the implementation of an embodiment of the method. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the devices, units and/or parts of at least one of the systems and/or products set forth.

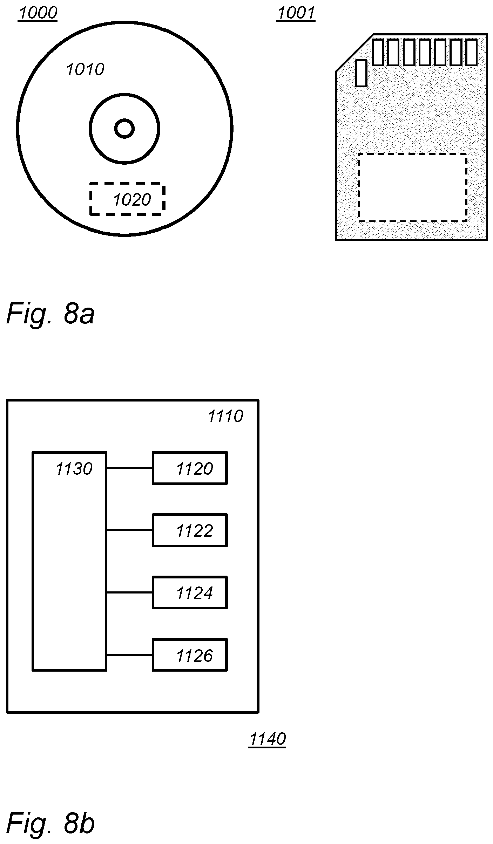

**Fig. 8a** shows a computer readable medium 1000 having a writable part 1010, and a computer readable medium 1001 also having a writable part. Computer readable medium 1000 is shown in the form of an optically readable medium.

Computer readable medium 1001 is shown in the form of an electronic memory, in this case a memory card. Computer readable medium 1000 and 1001 may store data 1020 wherein the data may indicate instructions, which when executed by a processor system, cause a processor system to perform an embodiment of a method for encoding or verifying genomic data, according to an embodiment. The computer program 1020 may be embodied on the computer readable medium 1000 as physical marks or by magnetization of the computer readable medium 1000. However, any other suitable embodiment is conceivable as well. Furthermore, it will be appreciated that, although the computer readable medium 1000 is shown here as an optical disc, the computer readable medium 1000 may be any suitable computer readable medium, such as a hard disk, solid state memory, flash memory, etc., and may be non-recordable or recordable. The computer program 1020 comprises instructions for causing a processor system to perform said method of encoding and/or verifying data.

**Fig. 8b** shows in a schematic representation of a processor system 1140 according to an embodiment of an encoding system and/or verifying system. The processor system comprises one or more integrated circuits 1110. The architecture of the one or more integrated circuits 1110 is schematically shown in Fig. 8b. Circuit 1110 comprises a processing unit 1120, e.g., a CPU, for running computer program components to execute a method according to an embodiment and/or implement its modules or units. Circuit 1110 comprises a memory 1122 for storing programming code, data, etc. Part of memory 1122 may be read-only. Circuit 1110 may comprise a communication element 1126, e.g., an antenna, connectors or both, and the like. Circuit 1110 may comprise a dedicated integrated circuit 1124 for performing part or all of the processing defined in the method. Processor 1120, memory 1122, dedicated IC 1124 and communication element 1126 may be connected to each other via an interconnect 1130, say a bus. The processor system 1110 may be arranged for contact and/or contact-less communication, using an antenna and/or connectors, respectively.

For example, in an embodiment, processor system 1140, e.g., the encoding or verification system may comprise a processor circuit and a memory circuit, the processor being arranged to execute software stored in the memory circuit. The memory circuit may be an ROM circuit, or a non-volatile memory, e.g., a flash memory. The memory circuit may be a volatile memory, e.g., an SRAM memory. In the latter case, the device may comprise a non-volatile software interface, e.g., a hard drive, a network interface, etc., arranged for providing the software.

While device 1140 is shown as including one of each described component, the various components may be duplicated in various embodiments. For example, the processor may include multiple microprocessors that are configured to independently execute the methods described herein or are configured to perform steps or subroutines of the methods described herein such that the multiple processors cooperate to achieve the functionality described herein. Further, where the device 1140 is implemented in a cloud computing system, the various hardware components may belong to separate physical systems. For example, the processor may include a first processor in a first server and a second processor in a second server.

The present invention includes the following further embodiments

Embodiment 1. An encoding system for encoding data in a data structure, the encoding system comprising

- an input interface configured for receiving the data,
- a processor system configured to

  - obtain the data as multiple data blocks,
  - compute multiple hash values for the multiple data blocks, by applying a hash function to the multiple data blocks,
  - compute a first hash tree for the multiple hash values, the multiple hash values being assigned to leaves of the first hash tree, one or more higher levels of the first hash tree being generated,
  - include in the data structure the multiple data blocks and whole or part of the first hash tree, said part excluding at least part of the leaves of the first hash tree.

Embodiment 2. An encoding system as in Embodiment 1, wherein the processor system is configured to compute a second hash tree, a root of the first hash tree being assigned to a leaf of the second hash tree, and multiple further hash values being assigned to multiple further leaves of the second hash tree, a further hash values being generated as a root of a further hash trees and/or generated by hashing on a further data block, and to include in the data structure at least the root of the second hash tree.

Embodiment 3. An encoding system as in any one of the preceding embodiments, wherein the data structure comprises data blocks organized in a hierarchy of containers, the processor system being configured to compute a hash tree for each container in the hierarchy, and each leaf of a hash tree being either the hash value of a data block within the container or the root of a hash tree that corresponds to a subordinate container.

Embodiment 4. An encoding system as in any one of the preceding embodiments, wherein the processor system is configured to compute a digital signature over a root of a hash tree, in particular the root of a hash tree having a hash tree root among its leaves, and to include the digital signature in the data structure.

Embodiment 5. An encoding system as in any one of the preceding embodiments, wherein the processor system is configured to store the data structure and/or stream the data structure or part of the data structure, said part including at least part of the data blocks and at least part of a hash tree corresponding to said data blocks.

Embodiment 6. An encoding system as in any one of the preceding embodiments, wherein a subset of the multiple data blocks and/or data containers are labelled as integrity protected and the rest of the multiple data blocks and/or data containers are labelled as integrity unprotected, only parts labelled as integrity protected being included in a hash tree.

Embodiment 7. An encoding system as in any one of the preceding embodiments, wherein the input interface is configured for receiving one or more tree parameters, the processor system being configured to include in the data structure a set of nodes selected from one or a hierarchy of hash trees depending on the one or more tree parameter.

Embodiment 8. An encoding system as in any one of the preceding embodiments, wherein the input interface is configured to receive amendments to the data, amendments including one or more of additions, deletions, and/or modifications, the processor system being configured to apply the amendments and to selectively recompute and update part of a hash tree corresponding to the amended part of the data.

Embodiment 9. An encoding system as in any one of the preceding embodiments,

- wherein a leaf of a hash tree further includes a hash of a data block in uncompressed form and a hash of the data block in compressed form, the data block being included in the data structure in compressed form; or
- wherein a leaf of a hash tree further includes a hash of a data block in uncompressed and unencrypted form, a hash of the data block in compressed and unencrypted form, a hash of a data block in compressed and encrypted form, the data block being included in the data structure in compressed and encrypted form.

Embodiment 10. A verification system for verifying selected data in a data structure, the verification system comprising

- an input interface configured for receiving at least part of the data structure, the data structure comprising multiple data blocks and part of a hash tree, excluding at least part of the leaves of the first hash tree,
- a processor system configured to

  - compute multiple hash values for data blocks in the data structure selected for data integrity verification, by applying a hash function to the selected data blocks,
  - identify a path starting from data blocks selected for verification to a root of a corresponding hash tree,
  - retrieve, if available in the data structure, or compute the hash values for the hash tree along the path,
  - verify the root of the hash tree from at least the computed multiple hash values.

Embodiment 11. A verification system as in Embodiment 10, wherein the data structure comprises a hierarchy of hash trees, the processor system being configured to identify a path starting from a leave to an overall root of the hierarchy of hash trees.

Embodiment 12. An encoding and/or verification system as in any one of embodiments 1-11, wherein the system is a device.

Embodiment 13. An encoding and/or verification system as in any one of embodiments 1-12, wherein the multiple data blocks comprise genomic data.

Embodiment 14. An encoding method for encoding data in a data structure, the encoding method comprising

- obtaining the data as multiple data blocks,
- computing multiple hash values for the multiple data blocks, by applying a hash function to the multiple data blocks,
- computing a first hash tree for the multiple hash values, the multiple hash values being assigned to leaves of the first hash tree, one or more higher levels of the first hash tree being generated,

- including in the data structure the multiple data blocks and part of the first hash tree, said part excluding at least part of the leaves of the first hash tree.

Embodiment 15. A verification method for verifying selected data in a data structure, the verification method comprising

- receiving at least part of the data structure, the data structure comprising multiple data blocks and part of a hash tree, excluding at least part of the leaves of the first hash tree,
- computing multiple hash values for data blocks in the data structure selected for data integrity verification, by applying a hash function to the selected data blocks,
- identifying a path starting from data blocks selected for verification to a root of a corresponding hash tree,
- retrieving, if available in the data structure, or compute the hash values for the hash tree along the path,
- verifying the root of the hash tree from at least the computed multiple hash values.

Embodiment 16. An encoding system for encoding genomic data in a digital data structure, the encoding system comprising

- an input interface configured for receiving the genomic data,
- a processor system configured to

  - obtain the genomic data as multiple data blocks,
  - compute multiple hash values for the multiple genomic blocks by applying a hash function at least to the genomic data in the multiple genomic data blocks,
  - compute a first hash tree for the multiple hash values, the multiple hash values being assigned to leaves of the first hash tree, the first hash tree having at least three levels,
  - include in the data structure the multiple genomic blocks and part of the first hash tree, said part including the first two highest levels of the first hash tree but excluding one or more lower levels of the first hash tree.

Embodiment 17. An encoding system as in any one of the preceding embodiments, wherein the processor system is configured to compute a second hash tree, a root of the first hash tree being assigned to a leaf of the second hash tree, multiple further data items being assigned to multiple further leaves of the second hash tree, and to include in the data structure at least the root of the second hash tree.

Embodiment 18. An encoding system as in any one of the preceding embodiments, wherein the data structure is a hierarchical data structure having multiple levels, the processor system being configured to compute a hash tree for each level of the hierarchical data structure, and to include in the hash trees for a level above the lowest level, a root of the hash tree computed for a lower level of the hierarchical data structure.

Embodiment 19. An encoding system as in any one of the preceding embodiments, wherein the input interface is configured for receiving a tree parameter, the processor system being configured to include in the data structure a larger or smaller part of the first hash tree depending on the tree parameter.

It should be noted that the above-mentioned embodiments illustrate rather than limit the presently disclosed subject matter, and that those skilled in the art will be able to design many alternative embodiments.
In the claims, any reference signs placed between parentheses shall not be construed as limiting the claim. Use of the verb 'comprise' and its conjugations does not exclude the presence of elements or steps other than those stated in a claim. The article 'a' or 'an' preceding an element does not exclude the presence of a plurality of such elements. Expressions such as "at least one of" when preceding a list of elements represent a selection of all or of any subset of elements from the list. For example, the expression, "at least one of A, B, and C" should be understood as including only A, only B, only C, both A and B, both A and C, both B and C, or all of A, B, and C. The presently disclosed subject matter may be implemented by hardware comprising several distinct elements, and by a suitably programmed computer. In the device claim enumerating several parts, several of these parts may be embodied by one and the same item of hardware. The mere fact that certain measures are recited in mutually different dependent claims does not indicate that a combination of these measures cannot be used to advantage.
In the claims references in parentheses refer to reference signs in drawings of exemplifying embodiments or to formulas of embodiments, thus increasing the intelligibility of the claim. These references shall not be construed as limiting the claim.

**Claims**

1. An encoding system for encoding data in a data structure, the encoding system comprising

   - an input interface configured for receiving the data,
   - a processor system configured to

      - obtain the data as multiple data blocks,
      - compute multiple hash values for the multiple data blocks, by applying a hash function to the multiple data blocks,
      - compute a first hash tree for the multiple hash values, the multiple hash values being assigned to leaves of the first hash tree, one or more higher levels of the first hash tree being generated,
      - include in the data structure the multiple data blocks and the whole or part of the first hash tree, said part excluding at least part of the leaves of the first hash tree, wherein the processor system is configured to compute a second hash tree, a root of the first hash tree being assigned to a leaf of the second hash tree, and multiple further hash values being assigned to multiple further leaves of the second hash tree, a further hash values being generated as a root of a further hash trees and/or generated by hashing on a further data block, and to include in the data structure at least the root of the second hash tree.

2. The encoding system as in claim 1, wherein the data structure comprises data blocks organized in a hierarchy of containers, the processor system being configured to compute a hash tree for each container in the hierarchy, and each leaf of a hash tree being either the hash value of a data block within the container or the root of a hash tree that corresponds to a subordinate container.

3. The encoding system as in any one of the preceding claims, wherein the processor system is configured to compute a digital signature over a root of a hash tree, in particular the root of a hash tree having a hash tree root among its leaves, and to include the digital signature in the data structure.

4. The encoding system as in any one of the preceding claims, wherein the processor system is configured to store the data structure and/or stream the data structure or part of the data structure, said part including at least part of the data blocks and at least part of a hash tree corresponding to said data blocks.

5. The encoding system as in any one of the preceding claims, wherein a subset of the multiple data blocks and/or data containers are labelled as integrity protected and the rest of the multiple data blocks and/or data containers are labelled as integrity unprotected, only parts labelled as integrity protected being included in a hash tree.

6. The encoding system as in any one of the preceding claims, wherein the input interface is configured for receiving one or more tree parameters, the processor system being configured to include in the data structure a set of nodes selected from one or a hierarchy of hash trees depending on the one or more tree parameter.

7. The encoding system as in any one of the preceding claims, wherein the input interface is configured to receive amendments to the data, amendments including one or more of additions, deletions, and/or modifications, the processor system being configured to apply the amendments and to selectively recompute and update part of a hash tree corresponding to the amended part of the data.

8. The encoding system as in any one of the preceding claims,

   - wherein a leaf of a hash tree further includes a hash of a data block in uncompressed form and a hash of the data block in compressed form, the data block being included in the data structure in compressed form; or
   - wherein a leaf of a hash tree further includes a hash of a data block in uncompressed and unencrypted form, a hash of the data block in compressed and unencrypted form, a hash of a data block in compressed and encrypted form, the data block being included in the data structure in compressed and encrypted form.

9. A verification system for verifying selected data in a data structure, the verification system comprising

   - an input interface configured for receiving at least part of the data structure, the data structure comprising multiple data blocks and part of a hash tree, excluding at least part of the leaves of the first hash tree,
   - a processor system configured to

- compute multiple hash values for data blocks in the data structure selected for data integrity verification, by applying a hash function to the selected data blocks,
- identify a path starting from data blocks selected for verification to a root of a corresponding hash tree,
- retrieve, if available in the data structure, or compute the hash values for the hash tree along the path,-
verify the root of the hash tree from at least the computed multiple hash values.

10. The verification system as in claim 9, wherein the data structure comprises a hierarchy of hash trees, the processor system being configured to

- identify a path starting from a leave to an overall root of the hierarchy of hash trees.

11. An encoding method (700) for encoding data in a data structure, the encoding method comprising

- obtaining (705) the data as multiple data blocks,
- computing (710) multiple hash values for the multiple data blocks, by applying a hash function to the multiple data blocks,
- computing (715) a first hash tree for the multiple hash values, the multiple hash values being assigned to leaves of the first hash tree, one or more higher levels of the first hash tree being generated,
- including (720) in the data structure the multiple data blocks and part of the first hash tree, said part excluding at least part of the leaves of the first hash tree,

wherein the method further comprises computing a second hash tree, a root of the first hash tree being assigned to a leaf of the second hash tree, and multiple further hash values being assigned to multiple further leaves of the second hash tree, a further hash values being generated as a root of a further hash trees and/or generated by hashing on a further data block, and to include in the data structure at least the root of the second hash tree.

12. A verification method (750) for verifying selected data in a data structure, the verification method comprising

- receiving (755) at least part of the data structure, the data structure comprising multiple data blocks and part of a hash tree, excluding at least part of the leaves of the first hash tree,
- computing (760) multiple hash values for data blocks in the data structure selected for data integrity verification, by applying a hash function to the selected data blocks,
- identifying (765) a path starting from data blocks selected for verification to a root of a corresponding hash tree,
- retrieving (770), if available in the data structure, or compute the hash values for the hash tree along the path,
- verifying (775) the root of the hash tree from at least the computed multiple hash values.

13. A verification method as in Claim 12, wherein the data structure comprises a hierarchy of hash trees, the method comprising identifying a path starting from a leave to an overall root of the hierarchy of hash trees.

14. A computer readable medium (1000) comprising data (1020) representing instructions, which when executed by a processor, cause the processor to perform the method according to claim 12 or 13.

110

130

140

150

*Fig. 1a*

160

170

180

190

*Fig. 1b*

200

A

HTA    SIG

220

230

240

210

213

A1

HTA1

A2

A3

HTA3

...

210

210

211

212

A11  A12  A13  ...  A14

A31  A32  A33  ...  A34

214

215

*Fig. 2a*

A

A1

A11  A12  A13  ...  A14  ...

A2

A3

...

...

A31  A32  A33  ...  A34

*Fig. 2b*

*Fig. 2c*

*Fig. 3*

400

410

420

430

440

*Fig. 4*

500

510

522

521

520

530

560

540

550

511

Fig. 5a

R

N31

N32

N21

N22

N23

N24

N11

N12

N13

N14

N15

N16

N17

N18

L1 L2 L3 L4 L5 L6 L7 L8 L9 L10 L11 L12 L13 L14 L15 L16

D1 D2 D3 D4 D5 D6 D7 D8 D9 D10 D11 D12 D13 D14 D15 D16

Fig. 5b

600

610 ——————————— 620

| | | 630 |
|---|---|---|
| 631 | 632 | 633 |

| | 640 |
|---|---|
| 641 | 642 |

| | 650 |
|---|---|
| 651 | 652 |

*Fig. 6*

*700* ⬎

```
┌──────────┐
│   705    │
└────┬─────┘
     │
     ▼
┌──────────┐
│   710    │
└────┬─────┘
     │
     ▼
┌──────────┐
│   715    │
└────┬─────┘
     │
     ▼
┌──────────┐
│   720    │
└──────────┘
```

*Fig. 7a*

*750* ⬎

```
┌──────────┐
│   755    │
└────┬─────┘
     │
     ▼
┌──────────┐
│   760    │
└────┬─────┘
     │
     ▼
┌──────────┐
│   765    │
└────┬─────┘
     │
     ▼
┌──────────┐
│   770    │
└────┬─────┘
     │
     ▼
┌──────────┐
│   775    │
└──────────┘
```

*Fig. 7b*

_1000_

_1001_

_1010_

_1020_

# Fig. 8a

_1110_

_1130_

_1120_

_1122_

_1124_

_1126_

_1140_

# Fig. 8b

Europäisches
Patentamt

European
Patent Office

Office européen
des brevets

# EUROPEAN SEARCH REPORT

Application Number

EP 21 20 7844

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BUCHMANN JOHANNES ET AL: "Long-term integrity protection of Check for updates of genomic data", EURASIP JOURNAL ON INFORMATION SECURITY, vol. 2019, no. 1, 29 October 2019 (2019-10-29), XP055790384, DOI: 10.1186/s13635-019-0099-x Retrieved from the Internet: URL:http://link.springer.com/article/10.11 86/s13635-019-0099-x/fulltext.html> * the whole document * | 1-14 | INV. G16B50/40 |
| A | BRADLEY TATIANA ET AL: "Genomic Security (Lest We Forget)", SECURITY & PRIVACY, IEEE, vol. 15, no. 5, 2017, pages 38-46, XP011661689, ISSN: 1540-7993, DOI: 10.1109/MSP.2017.3681055 [retrieved on 2017-10-02] * the whole document * | 1-14 | |
| A,D | US 4 309 569 A (MERKLE RALPH C) 5 January 1982 (1982-01-05) * column 2, line 16 - column 4, line 29 * | 1-14 | TECHNICAL FIELDS SEARCHED (IPC) G16B |

The present search report has been drawn up for all claims

| Place of search | Date of completion of the search | Examiner |
|---|---|---|
| Munich | 25 April 2022 | Díaz de Lezana, C |

EPO FORM 1503 03.82 (P04C01)

## ANNEX TO THE EUROPEAN SEARCH REPORT
## ON EUROPEAN PATENT APPLICATION NO.

**EP 21 20 7844**

This annex lists the patent family members relating to the patent documents cited in the above-mentioned European search report.
The members are as contained in the European Patent Office EDP file on
The European Patent Office is in no way liable for these particulars which are merely given for the purpose of information.

**25-04-2022**

| Patent document cited in search report | Publication date | Patent family member(s) | Publication date |
|---|---|---|---|
| US 4309569 A | 05-01-1982 | NONE | |

For more details about this annex : see Official Journal of the European Patent Office, No. 12/82

## REFERENCES CITED IN THE DESCRIPTION

**Patent documents cited in the description**

- US 4309569 A

**Non-patent literature cited in the description**

- Verkle Trees. **KUSZMAUL.** Technical report. Massachusetts Institute of Technology, 2018
- **BERNSTEIN.** *SPHINCS: practical stateless hash-based signatures*
- *GA4GH File Encryption Standard,* 21 October 2019